(19) Europäisches Patentamt
European Patent Office
Office européen des brevets

(11) Publication number: **0 006 718**
**B1**

(12) **EUROPEAN PATENT SPECIFICATION**

(45) Date of publication of patent specification: 30.03.83

(51) Int. Cl.³: C 07 D 249/12, A 61 K 31/41

(21) Application number: 79301141.2

(22) Date of filing: 14.06.79

(54) **1.2.4-Triazolidine-3.5-dione derivatives, process for their preparation and pharmaceutical compositions containing them.**

(30) Priority: 15.06.78 GB 2701178

(43) Date of publication of application: 09.01.80 Bulletin 80/1

(45) Publication of the grant of the patent: 30.03.83 Bulletin 83/13

(84) Designated Contracting States: BE CH DE FR GB IT NL SE

(56) References cited:
DE - A - 2 323 193
DE - A - 2 552 312
DE - A - 2 755 727
FR - A - 2 258 376

(73) Proprietor: BEECHAM GROUP PLC
Beecham House Great West Road
Brentford Middlesex (GB)

(72) Inventor: Adams, David Reginald
c/o Faculdad de Farmacia Universidad Central de Venezuela Caracas (VE)
Inventor: Barnes, Allen Frank
37 South Road
Bishops Stortford, Hertfordshire (GB)

(74) Representative: Stott, Michael John et al,
European Patent Attorney Beecham Pharmaceuticals Great Burgh Yew Tree Bottom Road
Epsom Surrey KT18 5XQ (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

# 1,2,4-Triazolidine-3,5-dione derivatives, process for their preparation and pharmaceutical compositions containing them

This invention relates to novel compounds having pharmacological activity, to a process for their preparation, and to pharmaceutical compositions containing them.

Offenlegungsschrift No: 2323193 discloses that pyrazolidine derivatives of the formula (I)':

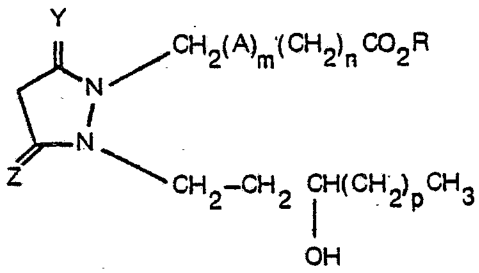

(I)'

wherein:

A is CH=CH or C≡C; R is H, an alkali metal, an amine salt, or a ≯12C hydrocarbon or chlorohydrocarbon residue; m is 0 or 1; n is 0—6; p is 0—6; and Y and Z are O or H except that Y and Z are not both O; have similar biological properties to the prostaglandins or are antagonists of prostaglandins.

French Patent Application No: 2258376 discloses that 10-aza prostaglandins of formula (II)'':

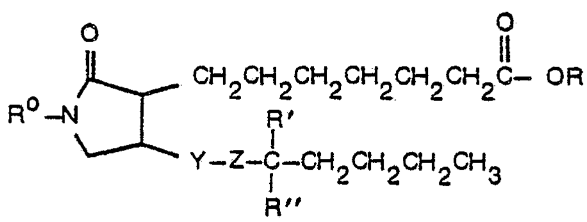

(II)''

wherein:

R=H or lower alkyl; R' and R''=$CH_3$ or $C_2H_5$; R°=H or lower alkyl; Y=—$CH_2$—$CH_2$—, or —CH=CH—; Z=—CO or —CH(~OH)—; are useful in the treatment of blood pressure and gastro-intestinal disorders, and in the preparation for confinement.

Belgian Patent No: 835989 discloses that compounds of the formula (III)'':

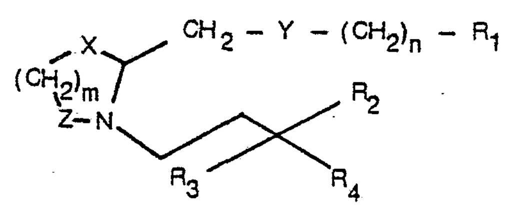

(III)''

wherein:

X is CO, protected CO, CROH in which R is hydrogen or $C_{1-4}$ alkyl and in which the OH moiety may be protected; Y is $CH_2CH_2$ or CH=CH; Z is CO or $CH_2$; n is 1 to 8; m is 1, 2 or 3; $R_1$ is hydrogen, $CH_2OH$, $CH_2OH$ in which the OH moiety is protected, $CO_2W$ wherein W is hydrogen or $CO_2W$ represents an ester group in which the ester moiety contains from 1 to 12 carbon atoms, or $CONH_2$; $R_2$ is hydrogen, $C_{1-4}$ alkyl, or taken together with $R_3$ and the carbon atom to which it is attached represents a carbonyl group; $R_3$ is hydrogen, hydroxy or protected hydroxy; $R_4$ is hydrogen or $C_{1-9}$ alkyl; and salts thereof; have useful pharmacological activity.

A novel class of compounds also having useful pharmacological activity has now been discovered, which compounds are structurally distinct from the prior art referred to above.

Accordingly the present invention provides a compound of the formula (I):

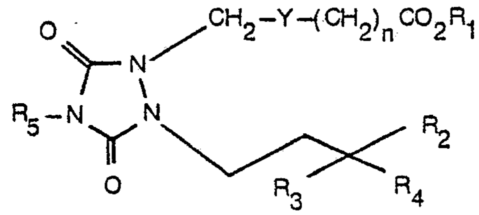

(I)

wherein:

n is 1 to 5
Y is $-CH_2-CH_2$, $-CH=CH-$ or $-C\equiv C-$
$R_1$ is hydrogen or $CO_2R_1$ represents an ester group in which the $R_1$ moiety contains from 1—12 carbon atoms;
$R_2$ is hydrogen, $C_{1-4}$ alkyl or phenyl;
$R_3$ is hydroxy or protected hydroxy;
$R_4$ is a group of the formula:

$$L-Q-R_{10}$$

in which:

Q is an oxygen atom, a $-CH=CH-$ group or a $-C\equiv C-$ group, and
L is a $C_{1-9}$ alkylene group; and
$R_{10}$ is $C_{1-4}$ alkyl, phenyl or naphthyl, any of which phenyl or naphthyl moieties may be substituted by one or more halogen, trifluoromethyl, $C_{1-6}$ alkyl, hydroxy, $C_{1-6}$ alkoxy, phenyl $C_{1-6}$ alkoxy or nitro groups, or, if Q is a $-CH=CH-$ group, then $R_{10}$ may be a hydrogen atom; and
$R_5$ is hydrogen or $C_{1-6}$ alkyl; and salts thereof.
Suitably n is 2, 3 or 4, preferably 3.
Similarly Y may be $-CH_2-CH_2-$ or $-CH=CH-$, suitably $-CH_2-CH_2-$.
$R_1$ is hydrogen, or $CO_2R_1$ represents an ester group in which the $R_1$ moiety contains from 1 to 12 carbon atoms. Examples of $R_1$ include hydrogen, methyl, ethyl, *n*- and *iso*-propyl, *n*-, *sec*- and *tert*-butyl, phenyl, benzyl and tolyl, while normally hydrogen or $C_{1-6}$ alkyl groups are preferred.

Suitable examples of $R_2$ include hydrogen, methyl, ethyl and phenyl. Preferred examples of $R_2$ include methyl.

Suitable protected hydroxy groups $R_3$ include readily hydrolysable derivatives such as acylated hydroxy groups in which the acyl moiety contains 1 to 4 carbon atoms, for example the acetoxy group; and hydroxy groups etherified by inert groups such as benzyl or methyl. Preferably however $R_3$ is hydroxy.

Within $R_4$, L may be straight chain alkylene groups such as *n*-propylene, *n*-butylene, *n*-pentylene, *n*-hexylene and *n*-heptylene or may be alkylene groups branched by one or two methyl groups at the same or different carbon atoms. $R_4$ groups more suitably have the structure $-(CH_2)_j-Q-R_{10}$, optionally substituted by one or two methyl groups at the same or different non-$R_{10}$ carbon atoms.

Within these more suitable groups $R_4$, when Q is an oxygen atom, j is 1 to 6. j is preferably 4 to 6, in particular 5, when $R_{10}$ is $C_{1-4}$ alkyl.

Within these more suitable groups $R_4$, when Q is a $-CH=CH-$ group or a $-C\equiv C-$ group, j is 1 to 4, preferably 1 or 2.

When $R_{10}$ is $C_{1-4}$ alkyl or a hydrogen atom subject to the proviso given under formula (I), then $R_{10}$ is preferably methyl or a hydrogen atom subject to the aforementioned proviso, so that specific examples of such $R_4$ groups are $-(CH_2)_5-O-Me$ and $-(CH_2)_2-CH=CH_2$.

When $R_{10}$ in $R_4$ is $C_{1-4}$ alkyl, it may suitably be straight chain, such as methyl, ethyl, *n*-propyl and *n*-butyl, or may suitably be branched by one or two methyl groups at the same or different carbon atoms, such as *iso*-propyl or *tert*-butyl; it is preferably methyl or ethyl.

When $R_4$ contains an aryl moiety as previously defined, these groups may be substituted in the phenyl or naphthyl moiety by normally one, two or three groups selected from those substituent groups listed hereinbefore. Examples of suitable substituent groups include fluorine, chlorine and bromine atoms and $CF_3$, methyl, ethyl, *n*- and *iso*-propyl, methoxy and ethoxy, *n*- and *iso*-propoxy and nitro groups. Other examples of such groups include hydroxy and benzyloxy. Preferably the aryl moieties when substituted by such groups will be mono or di-substituted.

Suitable examples of $R_5$ include hydrogen, methyl, ethyl, *n*- and *iso*-propyl, and *n*-, *sec* and *tert*-butyl.

Preferred $R_5$ groups include methyl.

The compounds of the formula (I) may form conventional salts when $R_1$ is hydrogen and also when $R_5$ is hydrogen.

Such salts include those with alkali and alkaline earth metals, suitably sodium and potassium, and ammonium and substituted ammonium salts.

Normally salts of the $R_5$ hydrogen will be with alkali metals.

One particularly suitable sub-group of compounds within formula (I) is of formula (II):

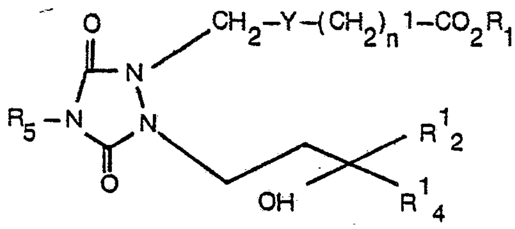

(II)

wherein:

Y, $R_1$ and $R_5$ are as defined in formula (I);
$n^1$ is 2, 3 or 4;
$R^1{}_2$ is hydrogen, methyl, ethyl or phenyl; and
$R^1{}_4$ is a group of the formula:

$$L—O—R^1{}_{10}$$

in which
L is a $C_{1-9}$ alkylene group; and
$R^1{}_{10}$ is $C_{1-4}$ alkyl;
and salts thereof.

Suitably in formula (II) $n^1$ is 3. Also Y is preferably $—CH_2CH_2—$.

Suitably $R_1$ is hydrogen or $C_{1-6}$ alkyl, preferably hydrogen.

Suitably $R^1{}_2$ is hydrogen, methyl or ethyl, preferably methyl.

Suitable and preferred straight chain and branched groups L include those previously described as suitable and preferred. Thus L is preferably a $C_{4-8}$ alkylene group.

Suitable and preferred $R^1{}_{10}$ are as so described for $R_{10}$ $C_{1-4}$ alkyl groups under formula (I).

$R_5$ is often methyl or ethyl, preferably methyl.

From the aforesaid it will be realised that one preferred group within formula (II) is of formula (III):

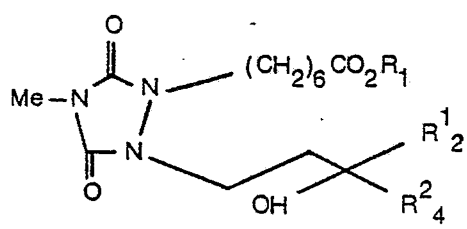

(III)

wherein:

$R_1$ is as defined in formula (I);
$R^1{}_2$ is hydrogen, methyl or ethyl; and
$R^2{}_4$ is a group of the formula:

$$L^1—O—R^2{}_{10}$$

in which
$L^1$ is a $C_{4-8}$ alkylene group; and
$R^2{}_{10}$ is $C_{1-2}$ alkyl;

and salts thereof.

Suitably $R_1$ in formula (III) is hydrogen or $C_{1-6}$ alkyl, preferably hydrogen. Suitable and preferred groups $L^1$ include those listed hereinbefore for L.

Another particularly suitable sub-group of compounds within formula (I) is of formula (IV):

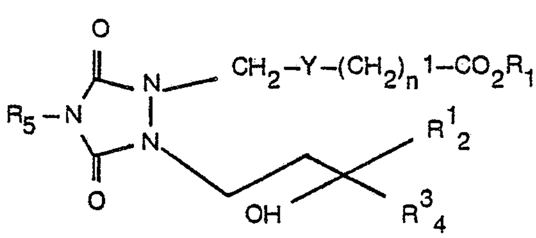

(IV)

wherein:

Y, $R_1$ and $R_5$ are as defined in formula (I);
$n^1$ is 2, 3 or 4;
$R^1_2$ is hydrogen, methyl, ethyl or phenyl; and
$R^3_4$ is a group of formula (V):

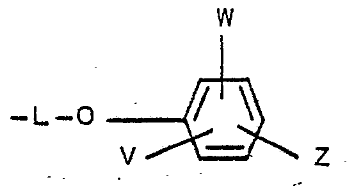

(V)

wherein:

L is as defined in formula (II); and V, W and Z are each hydrogen or fluorine, chlorine or bromine atoms, or $CF_3$, methyl, ethyl, *n* or *iso*-propyl, methoxy, ethoxy, *n*- or *iso*-propoxy or nitro groups; and salts thereof.

In formula (IV) it is preferred that $n^1$ is 3.
Suitably $R_1$ is hydrogen, sodium or $C_{1-6}$ alkyl, more preferably hydrogen.
V and W will often be hydrogen.
Preferred groups L are often those groups described as preferred under formula (I) for L.
Often $R_5$ will be methyl or ethyl, preferably methyl.
Another particularly suitable sub-group of compounds within formula (I) is of formula (VI):

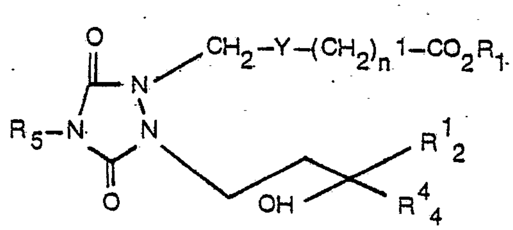

(VI)

wherein:

Y, $n^1$, $R_1$, $R^1_2$ and $R_5$ are as defined in formula (II); and
$R^4_4$ is a group of the formula

$$—L—Q^1—R^3_{10}$$

in which

L is as defined in formula (I);
$Q^1$ is a —CH=CH— group or a —C≡C— group;
$R^3_{10}$ is $C_{1-4}$ alkyl, or if $Q^1$ is a —CH=CH— group, then $R^3_{10}$ may be a hydrogen atom; and salts thereof.

Suitably in formula (VI) $n^1$ is 3.
Suitably $R_1$ is hydrogen or $C_{1-6}$ alkyl, preferably hydrogen.
Suitably $R^1_2$ is hydrogen, methyl or ethyl.
Suitable and preferred straight chain and branched groups L include those previously described as suitable and preferred. Thus L is preferably $C_{1-4}$ alkylene.
Suitable and preferred groups $R^3_{10}$ include hydrogen, methyl and ethyl.
Often in formula (VI) $R_5$ will be methyl or ethyl, preferably methyl.
From the aforesaid it will be relised that one preferred group within formula (VI) is of formula (VII):

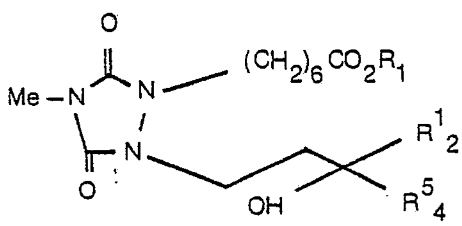

(VII)

wherein:

$R_1$ is as defined in formula (I);
$R^1_2$ is hydrogen, methyl or ethyl;
$R^5_4$ is a group of the formula

$L^3—Q^1—R^4_{10}$

in which

$L^3$ is a $C_{1-4}$ alkylene group;
$Q^1$ is a group —CH=CH— or a group —C≡C—;
$R^4_{10}$ is $C_{1-2}$ alkyl or may be hydrogen if $Q^1$ is a —CH=CH— group;

and salts thereof.

Suitably $R_1$ in formula (IX) is hydrogen or $C_{1-6}$ alkyl, preferably hydrogen.

Suitable and preferred groups $L^3$ include those listed hereinbefore for L.

Another particularly suitable sub-group of compounds within formula (I) is of formula (VIII):

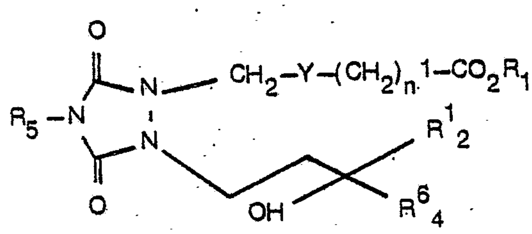

(VIII)

wherein:

Y, $R_1$ and $R_5$ are as defined in formula (I);
$n^1$ is 2, 3 or 4;
$R^1_2$ is hydrogen, methyl, ethyl or phenyl;
$R^6_4$ is a group of formula (IX):

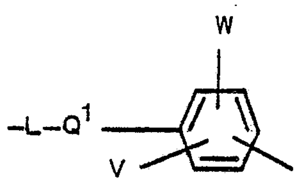

(IX)

wherein:

L and $Q^1$ are as defined in formula (VI); and
V, W and Z are each hydrogen or fluorine, chlorine or bromine atoms, or $CF_3$, methyl, ethyl, *n*- or *iso*-propoxy or nitro groups; and salts thereof.

In formula (VIII) it is preferred that $n^1$ is 3.

Suitably $R_1$ is hydrogen or $C_{1-6}$ alkyl, more preferably hydrogen.

Preferred L groups are as so described under formula (I). Also V and W will often be hydrogen.

Preferably $R_5$ is a $C_{1-6}$ alkyl group.

Often $R_5$ will be methyl or ethyl, preferably methyl.

Process variant a)

The invention also provides a process for the preparation of a compound of the formula (I), which process comprises reacting a compound of formula (X):

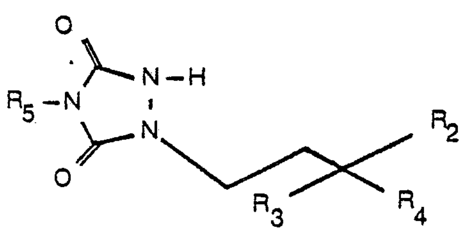

(X)

wherein:

$R_2$, $R_3$, $R_4$ and $R_5$ are as defined in formula (I), with a compound of formula (XI):

$$T—CH_2—Y—(CH_2)_nCO_2R_1 \qquad (XI)$$

wherein:

T is a group readily displaced by a nucleophile, and Y, n and $R_1$ are as defined in formula (I).

T is suitably a halogen, such as bromine, and the reaction suitably carried out in an organic solvent such as hexamethylphosphoramide in the presence of a base such as sodium carbonate. Generally it is preferred that $R_1$ in the compound of formula (XI) is other than hydrogen, and so if in this case a $R_1$ hydrogen compound of the formula (I) is desired it is prepared from the thus formed compound of the formula (I) by a conventional deesterification reaction. When $R_3$ is a protected hydroxy group in the compound of formula (X), then if in this case a compound of the formula (I) wherein $R_3$ is hydroxy is required it is prepared from the thus formed $R_3$ protected hydroxy compound by conventional de-protection reactions. For example, when $R_3$ is a benzyloxy group, the benzyl group may readily be removed by hydrogenolysis. Thus it can be seen that compounds of the formula (I) wherein $R_3$ is protected hydroxy are useful intermediates in the preparation of the corresponding free hydroxy compounds of the formula (I).

Process variant b)

The invention also provides a preferred process for the preparation of a compound of the formula (I), which process comprises reacting a compound of the formula (XII):

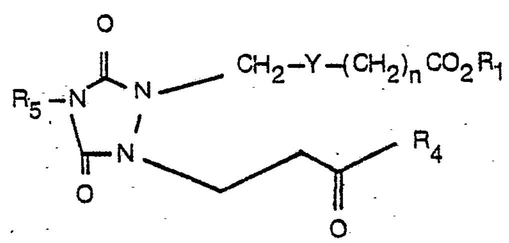

(XII)

wherein:

Y, n, $R_1$, $R_4$ and $R_5$ are as defined in formula (I); with a reducing agent to give a corresponding compound of the formula (I) wherein $R_2$ is hydrogen and $R_3$ is hydroxy, or with a $C_{1-4}$ alkyl or phenyl Grignard reagent or $C_{1-4}$ alkyl or phenyl metallic complex to give a corresponding compound of the formula (I) wherein $R_2$ is $C_{1-4}$ alkyl or phenyl, and $R_3$ is hydroxy; and then optionally protecting the $R_3$ hydroxy moiety.

The reduction of the side chain carbonyl in a compound of the formula (XII) may be carried out by conventional methods for reducing a ketone to an alcohol, for example by sodium borohydride reduction.

The $C_{1-4}$ alkyl or phenyl Grignard reagent or $C_{1-4}$ alkyl or phenyl metallic (suitably $C_{1-4}$ alkyl or phenyl lithium) complex reaction may be carried out under conventional conditions for such reactions, for example in an inert anhydrous solvent.

The optional protection of the $R_3$ hydroxy moiety may be carried out in conventional manner, for example by acylating, alkylating or benzylating the $R_3$ hydroxy compound.

Process variant c)

The invention also provides a further preferred process for the preparation of a compound of the formula (I), which process is convenient when $R_5$ in the compound of formula (I) is not a hydrogen atom, and which comprises reacting a compound of formula (XIII):

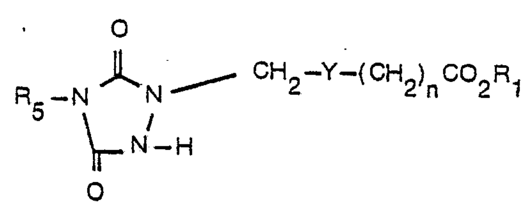

(XIII)

wherein:

Y, n, $R_1$ and $R_5$ are as defined in formula (I), with a compound of formula (XIV):

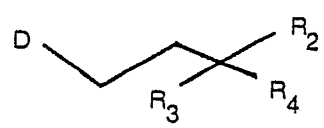

(XIV)

as defined above.

Suitable reaction conditions are those given hereinafter as suitable for the reaction of the compound of the formula (XIV) with the compound of the formula (XVII).

Suitable and preferred examples of D are those hereinafter described as suitable and preferred in the compounds of the formula (XIV).

After these reactions if so desired the group $R_1$ in the thus formed compounds of the formula (I) may be varied by conventional esterification and/or de-esterification reactions. Similarly when $R_1$ and/or $R_5$ is hydrogen in such compounds of the formula (I), salts of these compounds may be prepared in conventional manner, for example, by reacting the chosen compound of the formula (I) with the required base. Preferably strong bases such as sodium in an alcohol, e.g. ethanol, and similar reagents are used to obtain salts of $R_5$=H compounds.

When in the group $R_4$ Q is O and Y is —C≡C— or —CH=CH—. Y may be converted to the corresponding *cis*- CH=CH or $CH_2CH_2$ group by any usual reduction method such as palladium-catalysed hydrogenation.

The preparation of the intermediates for use in the processess of the invention will now be described.

Process variant a), Intermediates

Compounds of the formula (X) may be prepared by reacting a compound of the formula (XVI):

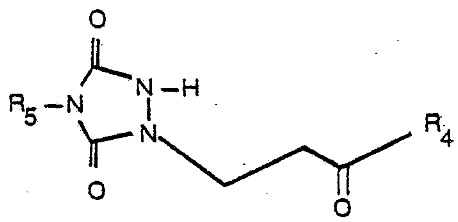

(XVI)

with a reducing agent to give a corresponding compound of the formula (X) wherein $R_2$ is hydrogen and $R_3$ is hydroxy.

The reduction may be carried out as hereinbefore described with reference to compounds of the formula (XII).

Compounds of the formula (X) may also be prepared by reacting a compound of the formula (XVII):

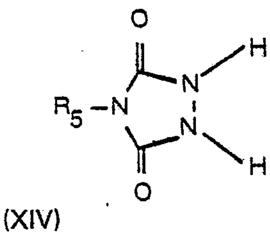

(XVII)

with a compound of the formula (XIV)

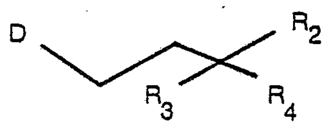

(XIV)

wherein:

D is a good leaving group, and
$R_2$, $R_3$ and $R_4$ are as defined in formula (I).

This reaction is suitably carried out in an inert organic solvent, such as hexamethylphosphoramide or N,N - dimethyl - formamide, at room temperature, in the presence of a base, such as sodium carbonate or sodium hydride, and a source of alkali metal ions, such as an alkali metal halide. Suitable alkali halides include sodium iodide and lithium iodide.

Suitable examples of D include tosylate, bromide or iodide. Preferably D is a tosylate residue.

Compounds of the formula (XI) are either known compounds or may be prepared by a process analogous to those used for preparing known compounds.

The compound of formula (X) may also be prepared by cyclising a compound of the formula (XIX)

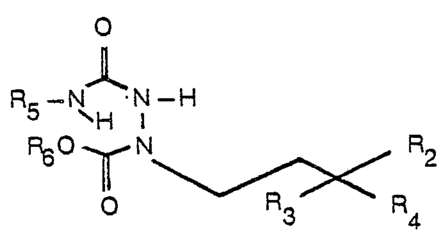

(XIX)

wherein $R_2$, $R_3$, $R_4$ and $R_5$ are as defined in formula (I) and $R_6$ is an organic group of up to 12 carbon atoms.

Suitable groups $R_6$ include $C_{1-6}$ alkyl.

The reaction is suitably carried out in an inert organic solvent, such as benzene or toluene at room temperature in the presence of a strong organic base such as an alkali metal alkoxide, for example potassium *tert*-butoxide, or a polycyclic aza-substituted base, such as DBU (diazabicycloundecene).

When $R_5$ is not a hydrogen atom, the compound of formula (XIX) is conveniently prepared, and subsequently cyclised *in situ*, by the reaction of a compound of formula (XX):

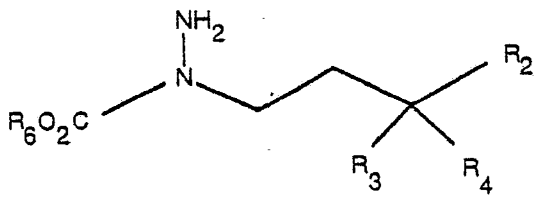

(XX)

with a compound $R_5NCO$, conveniently in an inert organic solvent such as toluene, under reflux.

The compound of formula (XX) may be prepared by the N-nitrosylation and reduction of a compound of the formula (XXI) in conventional manner:

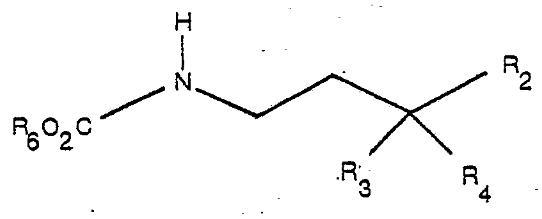

(XXI)

One suitable reaction scheme for the preparation of compounds of the formula (XXI) is shown below:

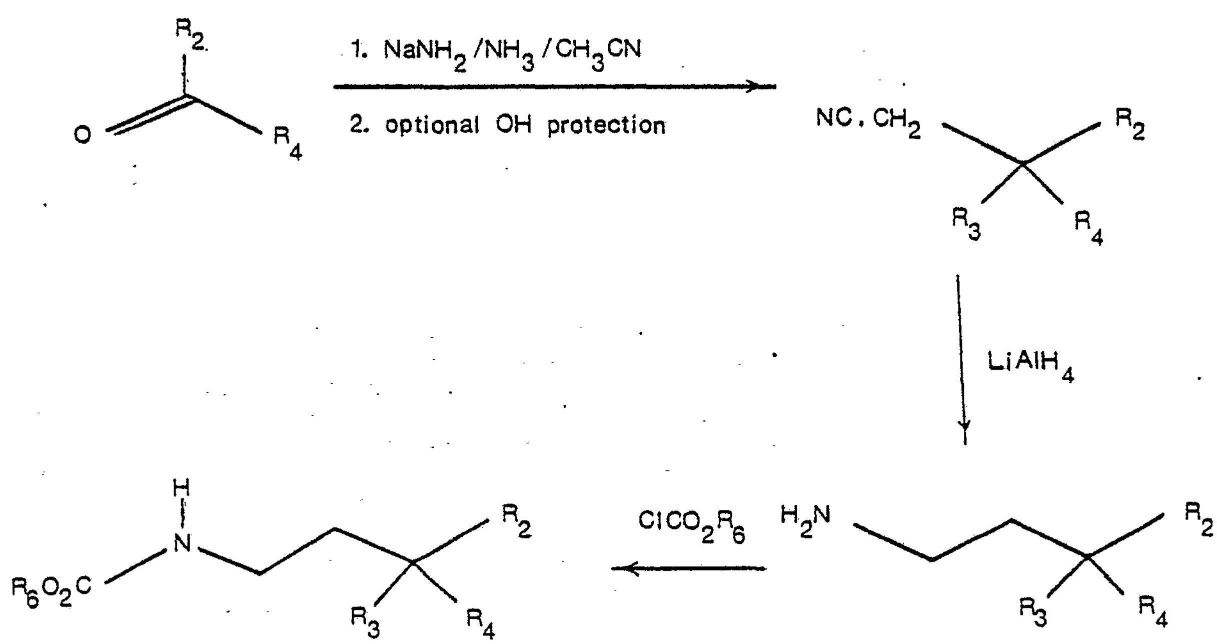


(XXI)

Process variant b), Intermediates

Compounds of the formula (XII) may be prepared by a process which comprises reacting a compound of the formula (XVI) as defined with a compound of the formula (XI) as defined.

Compounds of the formula (XVI) may be prepared by reacting a compound of the formula (XVII):

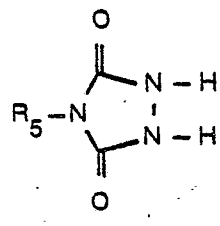

(XVII)

with a strong base and a compound of formula (XV):

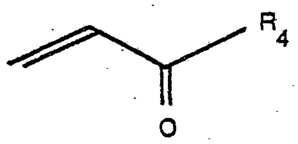

(XV)

Compounds of the formula (XVII) are either known compounds or can be prepared in analogous manner to known compounds.

For example, one suitable reaction scheme for the preparation of these compounds is shown below:

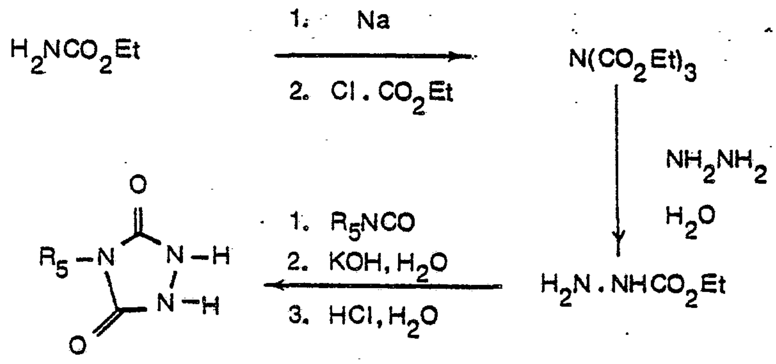


Process variant c), Intermediates

When $R_5$ is not a hydrogen atom, the compound of formula (XIII) wherein Y is *trans*- CH=CH or —$CH_2CH_2$ may be prepared by reacting a compound of formula (XVIII):

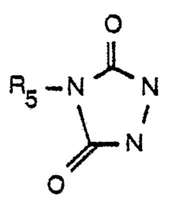

(XVIII)

with a compound of formula 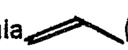 $(CH_2)_{n+1}$ $CO_2R_1$, and then if necessary reducing the resulting Y is CH=CH compound to the corresponding Y is $CH_2CH_2$ compound.

This reaction is suitably carried out in an inert organic solvent, such as benzene, at the reflux temperature, under an inert atmosphere. It should be mentioned that to prepare a compound of the formula (XIII) wherein $R_1$ is other than hydrogen, it is generally preferred to prepare the corresponding compound of the formula (XIII) wherein $R_1$ is hydrogen and then to convert that compound to the desired compound by conventional methods. For example a $R_1$ is hydrogen compound may be converted to a $R_1$ is methyl compound by treatment with acetyl chloride in methanol.

The optional reduction can be carried out in conventional manner.

When $R_5$ is not a hydrogen atom, the compound of formula (XVIII) may be prepared by treating a compound of formula (XVII) as hereinbefore defined, that is, a compound of the formula:

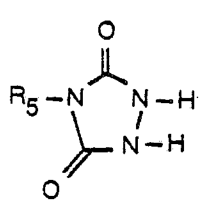

(XVII)

with an oxidising agent, such as $N_2O_4$ or *t*-butyl hypochlorite.

This reaction is suitably carried out by suspending the chosen compound of the formula (XVII) in an inert organic solvent, such as dichloromethane, at 0°C, and bubbling $N_2O_4$ through this suspension, or adding a known volume of $N_2O_4$ in dichloromethane slowly to the suspension.

The preparation of the compounds of the formula (XVII) has been discussed hereinbefore.

It is believed that compounds of formula (X), (XII), (XIII) and (XVI) are novel compounds, and these compounds are useful intermediates as hereinbefore described.

It will be realised by the skilled reader that although the reaction sequences leading to the active compounds of the invention hereinbefore described are particularly suitable, a number of variations in the sequences are possible. It is believed these variations are best illustrated by use of the following flow diagram.

(The reactions represented by arrows in the flow diagram are carried out as hereinbefore described, or in an analogous manner).

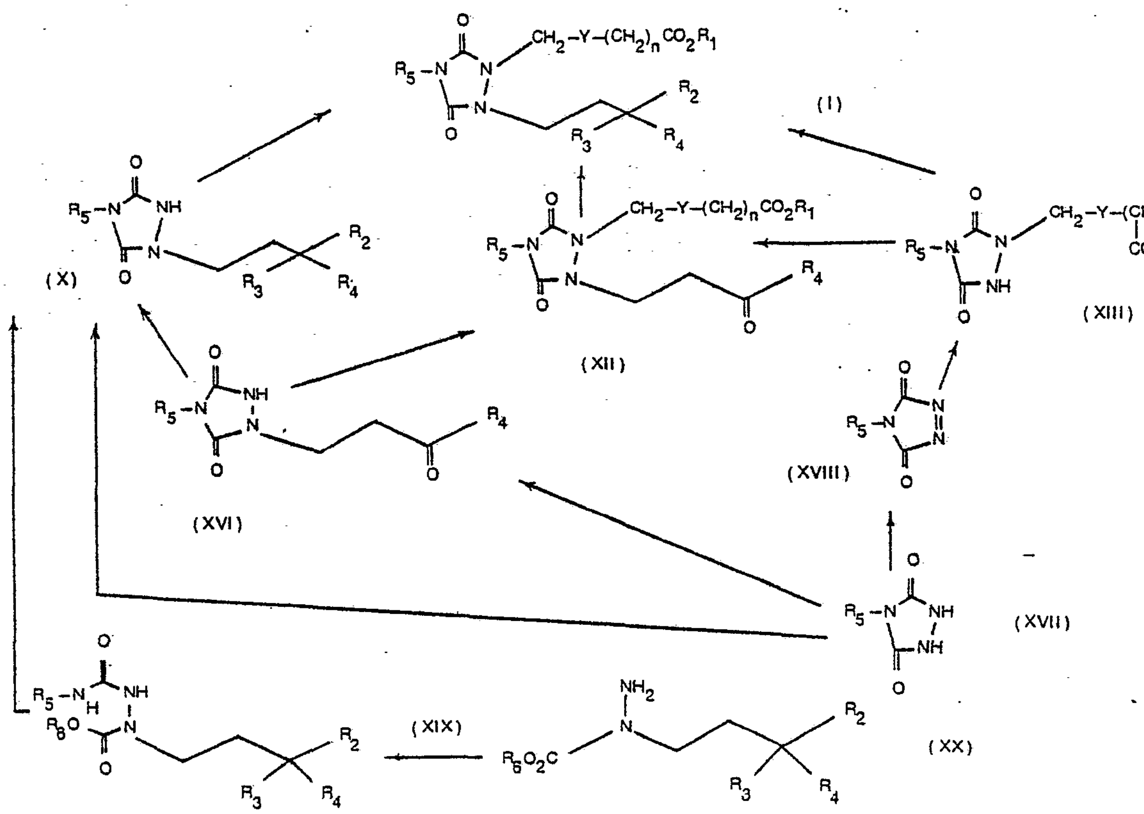
(I) (X) (XII) (XIII) (XVI) (XVIII) (XVII) (XIX) (XX)

It will of course be realised that the compounds of the formula (I) have an asymmetric centre, and thus are capable of existing in enantiomeric forms. The invention extends to each of these isomeric forms, and to mixtures thereof. The different isomeric forms may be resolved by the usual methods.

Compounds within the formula (I) have useful pharmacological activity. For example compounds within the formula (I) have anti-gastric secretion activity e.g. anti-ulcer activity, cardiovascular activity e.g. anti-hypertensive activity or anti-arrhythmic activity, platelet aggregation inhibition activity, affect the respiratory tract e.g. bronchodilator activity, and have anti-fertility and smooth muscle activity.

In general it may be said that compounds within the formula (I) have a range of pharmacological activities similar to those shown by the natural prostaglandins, but that these activities tend to be rather more selective.

Compounds of the formula (I) wherein Q in the group $R_4$ is an oxygen atom include compounds which in particular exhibit anti-gastric secretion activity, e.g. anti-ulcer activity, especially those compounds of formula (IV).

The invention therefore also provides a pharmaceutical composition comprising a compound of the formula (I) and a pharmaceutically acceptable carrier.

Clearly the formulation of the said pharmaceutical composition will depend on the nature of the activity shown by the chosen compound of the formula (I), and on other factors such as preference in a particular area of therapy for a particular mode of administration.

The compositions may be in the form of tablets, capsules, powders, granules, lozenges or liquid preparations, such as oral or sterile parenteral solutions or suspensions.

Tablets and capsules for oral administration may be in unit dose presentation form, and may contain conventional excipients such as binding agents, fillers, tabletting lubricants, disintegrants and

acceptable wetting agents. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contains conventional additives such as suspending agents, emulsifying agents, non-aqueous vehicles (which may include edible oils), preservatives, and if desired conventional flavouring or colouring agents.

For parenteral administration, fluid unit dosage forms may be prepared utilizing the compound of the formula (I) and a sterile vehicle. The compound, depending on the vehicle and concentration used, can be either suspended or dissolved in the vehicle. In preparing solutions the compound can be dissolved for injection and filter sterilized before filling into a suitable vial or ampoule and sealing. Advantageously, adjuvants such as a local anaesthetic, preservatives and buffering agents can be dissolved in the vehicle. Parenteral suspensions are prepared in substantially the same manner except that the compound is suspended in the vehicle instead of being dissolved and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

When appropriate, the compositions of this invention may be presented as an aerosol for oral administration, or as a microfine powder for insufflation.

As is common practice, the compositions will usually be accompanied by written or printed directions for use in the medical treatment concerned.

It will of course be realised that the precise dosage used in the treatment of any of the hereinbefore described disorders will depend on the actual compound of the formula (I) used, and also on other factors such as the seriousness of the disorder being treated.

The following Examples 4, 5 and 8 illustrate the preparation of the invention.

The following Examples 1 to 3, 6 and 7 illustrate the preparation of intermediates therefor.

Example 1

Preparation of 1-(p-Toluenesulphonyloxy)-3-methyl-4-phenoxy-butan-3-ol

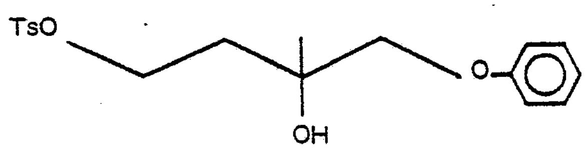

To a slurry of zinc (33 g, 0.5 mol) in benzene (50 ml) containing a small crystal of iodine and under reflux with stirring, was added carefully 40 ml of a mixture of phenoxy - propan - 2 - one (44 g, 0.29 mol), ethylbromoacetate (84 g, 0.5 mol) and benzene (50 ml) after reaction initiation the rest of the mixture was added at such a rate that reflux was maintained. After complete addition of the ketone mixture, the resultant reaction mixture was boiled at reflux for a further 1 hr. The mixture was then cooled and poured into ice-cold 20% sulphuric acid (200 ml) and extracted with diethyl ether (4×200 ml). The combined extracts were washed with water (2×250 ml), saturated sodium hydrogen carbonate solution (2×250 ml) and saturated sodium chloride solution until neutral. The extract was then dried ($Na_2SO_4$), filtered and the filtrate concentrated *in vacuo* to leave an oil (65 g). This residual oil was then distilled to afford ethyl - 3 - hydroxy - 3 - methyl - 4 - phenoxybutyrate (35.3 g) b.p. 133—7°C/(0.05 mm. Hg) 6.67 Pa.

To a slurry of lithium aluminium hydride (5.64 g, 0.148 mol) in diethyl ether (250 ml) under nitrogen and cooled in an ice bath was added dropwise ethyl - 3 - hydroxy - 3 - methyl - 4 - phenoxybutyrate (35.3 g, 0.148 mol) in diethyl ether (100 ml). After complete addition of the ester solution the resultant mixture was boiled at reflux for 1 hr, then cooled in an ice-bath. Excess lithium aluminium hydride was destroyed by successive dropwise addition of water (6 ml), 10% sodium hydroxide solution (6 ml) and water (18 ml). The reaction mixture was filtered, the filter cake washed with diethyl ether (2×100 ml), and the filtrate washed with saturated sodium chloride solution (1×250 ml) and then dried ($Na_2SO_4$). This mixture was filtered and the filtrate concentrated *in vacuo* to give 3 - methyl - 4 - phenoxybutane - 1,3 - diol (26.5 g) as an oil.

N.m.r. ($CDCl_3$) $\tau$: 2.68—3.38 bm, 5H, $C_6H_5$—; 5.82—6.42 m, 6H, $CH_2$—OH, $PhOCH_2$, OH×2; 8.02—8.38 m, 2H, $CH_2$; 8.73 s, 3H, $CH_3$.
I.r ($cm^{-1}$): 3500, OH; 1603, Ar.

The 3 - methyl - 4 - phenoxybutane - 1,3 - diol (26.5 g, 0.135 mol) was dissolved in pyridine (150 ml) and cooled to 0°C with stirring p-toluenesulphonyl chloride (27 g, 0.142 mol) was added portionwise, and the resultant mixture was stirred for 45 minutes at 0°C. The reaction mixture was stored in a refrigerator for 15 hr and then poured into iced-water (200 ml). The reaction mixture was extracted with diethyl ether (3×200 ml) and the combined extracts washed with 10% hydrochloric acid

(2×200 ml), saturated sodium chloride solution (3×200 ml) and then dried ($Na_2SO_4$). This mixture was filtered and the filtrate concentrated *in vacuo* at room temperature to leave a gum (38 g). This gum was mixed with petrol (bp 60—80°C, 200 ml), gradually cooled to −78°C with stirring and the petrol decanted. Residual petrol was removed by evaporation *in vacuo* at room temperature to leave *1 - (p - toluenesulphonyloxy) - 3 - methyl - 4 - phenoxybutan - 3 - ol* (38 g) as a gum.

| | |
|---|---|
| Analysis: | C, 62.01; H, 6.49; S, 9.53%. |
| | $C_{18}H_{22}O_5S$ requires: C, 61.71; H, 6.33; S, 9.13%. |
| N.m.r. ($CDCl_3$) $\tau$: | 2.23—3.32 m, 9H, aromatic; 5.81 t, 2H, $CH_2$—OTs; 6.35 s, 2H $PhOCH_2$; 7.23 bs, 1H, —$OH$; 7.64 s, 3H, 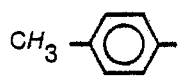; 8.08 t, 2H, $CH_2$—$CH_2$—OT s; 8.78 s, 3H, $CH_3$. |
| I.r ($cm^{-1}$): | 3600, —OH; 1603, aromatic; 1190, 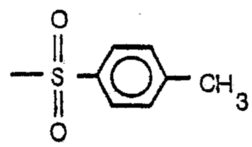 |

Example 2
Preparation of 4,4-dimethyl-8-methoxy-oct-1-en-3-one

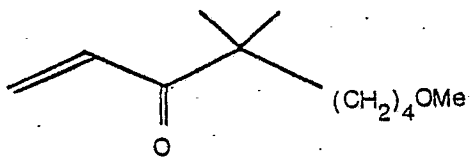

Sodium (53.2 g, 2.32 g. atom) was dissolved in methanol (500 ml) and the resultant solution was added dropwise, with stirring, to 1,4 - dibromobutane (500 g, 2.32 mol) at 60°C. The resultant mixture was boiled at reflux for 6 hr, cooled and filtered. The filtrate was poured into water (1200 ml) and extracted with diethyl ether (4×600 ml). The combined extracts were washed with water (2×300 ml), dried ($Na_2SO_4$), filtered and concentrated *in vacuo*. The residue was fractionally distilled to afford 1 - bromo - 4 - methoxybutane (65.1 g) bp. 52—60°C, (15 mm. Hg.) 2 kPa.

| | |
|---|---|
| N.m.r. ($CDCl_3$) $\tau$: | 6.50—6.73 m, 6.73 s, 7H, $BrCH_2(CH_2)_2CH_2OCH_3$; 7.80—8.50 m, 4H, $BrCH_2(CH_2)_2CH_2OCH_3$. |

To ice-cold cyclohexylamine (198 g, 2.0 mol) was added dropwise, with stirring, isobutyraldehyde (144 g, 2.0 mol) and the mixture stirred at 0°C for 3 hr. To the reaction mixture was then added potassium carbonate (100 g) and benzene (200 ml) and the resultant mixture stirred overnight at room temperature. The mixture was then filtered and the benzene removed by distillation at atmospheric pressure. The residual oil was then distilled to afford N - (2 - methyl - propylidene)cyclohexylamine (320 g) bp 81—5°C/(20 mm. Hg) 2.67 kPa. (ref bp 82°C/(26 mm hg.) 3.47 kPa). To a solution of lithium diethylamide prepared from lithium (2.98 g, 0.43 mol) and diethylamine (32.0 g, 0.43 mol), in hexamethylphosphoramide (92 ml), benzene (84 ml) and tetrahydrofuran (80 ml) cooled to −78° under nitrogen, was added dropwise, with stirring N - (2 - methylpropylidene)cyclohexylamine (68 g, 0.39 mol) in tetrahydrofuran (80 ml). The resultant mixture was allowed to warm to 0° during 0.5 h and was then re-cooled to −78°C and stirred for a further 1 h at −78°C. To this reaction mixture was added dropwise 1 - bromo - 4 - methoxybutane (65.1 g, 0.39 mol) in tetrahydrofuran (80 ml) and the resultant solution was allowed to warm to room temperature and stirred at this temperature for 2 hr. Water (100 ml) followed by 5N hydrochloric acid (20 ml) were then added, and the mixture was boiled at reflux for 0.5 hr. The mixture was then cooled and extracted with diethyl ether (4×200 ml). The combined extracts were washed with 10% hydrochloric acid (2×200 ml), water (2×200 ml) and saturated sodium chloride solution until neutral, and then dried ($Na_2SO_4$) and filtered, and the filtrate was concentrated *in vacuo*, to afford an oil (84 g). This residual oil was distilled to afford 2,2 - di - methyl - 6 - methoxyhexanal (36 g) bp. 84—92°C/(15 mm. Hg) 2 kPa.

| | |
|---|---|
| N.m.r. ($CDCl_3$) $\tau$: | 0.66, 1H, —$CHO$; 6.58—6.78 m, 6.78 s, 5H, $CH_2$—$OCH_3$; 8.4—8.78 m, 6H, $(CH_2)_3$; 8.99 S, 6H, $(CH_3)_2$. |

To vinyl magnesium bromide prepared from magnesium (7.45 g, 0.31 g atom), vinyl bromide (36.9 g 0.34 mol) in tetrahydrofuran (140 ml), was added dropwise, with stirring 2,2 - di - methyl - 6 - methoxyhexanal (36 g, 0.23 mol) in tetrahydrofuran (60 ml), and the resultant mixture was stirred for 15 hr at room temperature. The reaction mixture was poured into saturated ammonium chloride solution (200 ml) and extracted with diethyl ether (4×200 ml). The combined extracts were washed with saturated sodium chloride solution (2×250 ml) and then dried ($Na_2SO_4$) and concentrated *in vacuo*. The residual oil was distilled to afford 4,4 - dimethyl - 8 - methoxy - oct - 1 - en - 3 - ol (31.7 g) bp 120—127°C, (14 mm Hg.) 1.87 kPa.

N.m.r. ($CCl_4$) $\tau$: 3.85—4.40 m, 1H; 4.70—5.10 m, 2H; 6.32 d, 1H, *CH*—OH; 6.6—6.8 m, 6.8 s, 5H, $CH_2$—O—$CH_3$; 7.78 bs, 1H, —*OH*; 8.45—8.93 bm, 6H, 2×$CH_3$, 9.23 d, 6H, $(CH_3)_2$.

To a solution of 5,5 - dimethyl - 8 - methoxy - oct - 1 - en - 3 - ol (31.7 g, 0.17 mol) in acetone (200 ml) at 0°C was added, dropwise during 2 hr, a solution of chromium trioxide (17.5 g, 0.17 mol) in water (52 ml) containing concentrated sulphuric acid (15 ml). The resultant solution was stirred for a further 0.5 hr, then poured into water (100 ml) and extracted with diethyl ether (4×200 ml). The combined extracts were washed with saturated sodium chloride solution (4×200 ml), dried ($Na_2SO_4$) and 10 mg hydroquinone added. The extracts were then filtered and the filtrate concentrated *in vacuo*. The residual oil was distilled to afford 5,5 - dimethyl - 8 - methoxy - oct - 1 - en - 3 - one (15 g), bp 112—124°C, (14 mm Hg) 1.87 kPa.

N.m.r. ($CDCl_3$) $\tau$: 3.15 q, J=9, 17 Hz; 3.70 q, J=3, 17 Hz; 4.37 q, J=3, 9 Hz; 6.5—6.69 bm, 6.69 s, 5H; 8.17—8.84 m, 8.84 s, 12H.
I.r ($cm^{-1}$): 1685, C=O; 1610, C=C.

Example 3
Preparation of 1-(6'-methoxycarbonyl-n-hexyl)-4-methyl-1,2,4-triazolidine-3,5-dione

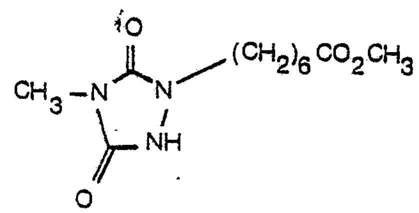

Dinitrogen tetroxide was bubbled through a suspension of 4 - methyl - 1,2,4 - triazolidine - 3,5 - dione[2] (23.0 g, 0.2 mol) in dichloromethane (250 ml) cooled to 0° until a clear, homogeneous deep red solution was obtained. This solution was then dried ($Na_2SO_4$) and filtered, and the filtrate was evaporated at room temperature *in vacuo* to give 4 - methyl - 1,2,4 - triazoline - 3,5 - dione (mp 105°C, decomp.). To the triazoline (23.0 g, 0.2 mol) dissolved in benzene (200 ml) was added dropwise, with stirring, hept - 6 - enoic acid (23.0 g, 0.18 mol) in benzene (100 ml) and the resultant solution boiled under reflux in an atmosphere of nitrogen for 1 hr. The reaction mixture was cooled and evaporated *in vacuo*. The residue was dissolved in a 10% solution of acetyl chloride in methanol (300 ml) and boiled at reflux for 5 hr and then left at room temperature overnight. The solution was evaporated and the residue (44 g) was chromatographed on silica gel (Merck Kieselgel 60, 900 g) using chloroform: methanol (0.5%) as eluant to afford 1 - (6' - methoxycarbonyl - n - hex - 2 - enyl) - 4 - methyl - 1,2,4 - triazolidine - 3,5 - dione (19.1 g) m.p. 55—7°C.

Analysis: C, 51.55; H, 6.56; N, 16.59%
$C_{11}H_{17}N_3O_4$ requires C, 51.76; H, 6.71; N, 16.46%.
N.m.r. ($CDCl_3$) $\tau$: 0.8—1.2 bs, 1H, N—H; 4.37 m, 2H, *CH*=*CH*; 5.9 s, bd, 2H, N—$CH_2$—; 6.40 s, 3H, —$CO_2CH_3$; 7.00 S, 3H, N—$CH_3$; 7.5—8.8 bm, 6H, —$(CH_2)_3$.
I.r ($cm^{-1}$): 1760, —N—$\underset{\underset{O}{\|}}{C}$—; 1670—1740, N—$\underset{\underset{O}{\|}}{C}$—, $CO_2Me$.

Mass Spec. Meas. mass 255. 1243; calc. mass 255. 1219.

The 1 - (6' - methoxycarbonyl - n - hex - 2 - enyl) - 4 - methyl - 1,2,4 - triazolidine - 3,5 - dione (12.4 g, 0.049 mol) was dissolved in dimethoxyethane (200 ml), 10% palladium on charcoal (3 g) was added, and the resultant mixture allowed to take up hydrogen (ca. 1100 ml). After the reaction was complete the resultant mixture was filtered through a Kieselguhr bed and the filtrate evaporated *in vacuo* to afford 1 - (6' - methoxycarbonyl - n - hexyl) - 4 - methyl - 1,2,4 - triazolidine - 3,5 - dione (12.3 g) mp 80—1°C.

Analysis found: C, 51.12; H, 7.71; N, 16.38%.
$C_{11}H_{19}N_3O_4$ requires C, 51.35; H, 7.44; N, 16.33%.

N.m.r. ($CDCl_3$) $\tau$: 6.39 S, 6.48 m, 5H, $CO_2CH_3$; N—$CH_2$; 6.97 S, 3H, N—$CH_3$; 7.73 m, 2H, $CH_2CO_2Me$; 9.07—9.78 bm, 8H, $(CH_2)_4$.

O
||
I.r (Nujol, $cm^{-1}$): 1660—1780, N—C—, $CO_2Me$.

Mass Spec: Meas. mass 257. 1377; calc. mass 257. 1375.

Example 4

Preparation of 1-(6′-carboxyhexyl)-2-(3″-hydroxy-3″-methyl-4″-phenoxybutyl)-4-methyl-1,2,4-triazolidine-3,5-dione

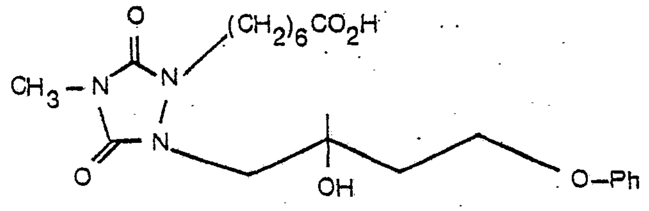

To a solution of 1 - (6′ - methoxycarbonyl - n - hexyl) - 4 - methyl - 1,2,4 - triazolidine - 3,5 - dione (2.0 g, 0.008 mol) in dimethylformamide (20 ml) was added portionwise sodium hydride as an 80% dispersion in mineral oil (0.26 g, 0.009 mol). After 0.5 h and 50°C under nitrogen, lithium iodide (2.3 g, 0.018 mol) and a solution of 1 - (p - toluenesulphonyloxy) - 3 - methyl - 4 - phenoxybutan - 3 - ol (2.73 g, 0.008 mol) in dimethylformamide (20 ml) were added and the resultant mixture stirred for 15 hr at 50°C.

The reaction mixture was then cooled and poured into water (200 ml) and after acidification with 10% hydrochloric acid, the resultant mixture was extracted with ethyl acetate (3×200 ml). The combined extracts were washed with saturated sodium chloride solution (3×250 ml), dried ($Na_2SO_4$) and then filtered, and the filtrate was concentrated to give a gum (4.24 g). This gum was chromatographed on silica gel (200 g, Merck Kieselgel 60) using chloroform as eluant to afford 1 - (6′ - methoxycarbonyl - n - hexyl) - 2 - (3″ - hydroxy - 3″ - methyl - 4″ - phenoxy - n - butyl) - 4 - methyl - 1,2,4 - triazolidine - 3,5 - dione (1.37 g) as a gum.

Analysis: C, 60.42; H, 7.59; N, 9.79%.
$C_{22}H_{33}N_3O_6$ requires: C, 60.67; H, 7.64; N, 9.65%

N.m.r. ($CDCl_3$) $\tau$: 2.5—3.2 m, 5H, $C_6H_5$; 6.0—6.77 m, 6.15 S, 6.31 S, 9H, $(N—CH_2)_2$, $CH_2$—OPh, $CO_2CH_3$; 6.94 S, 3H, N—$CH_3$; 7.26 bs, 1H, —OH; 7.70 bt, 2H, —$CH_2CO_2Me$; 7.90—9.0 m, 8.64 S, 13H.

O O
|| ||
I.r ($cm^{-1}$): 3500, OH; 1770, N—C—; 1680—1760, N—C, $CO_2Me$; 1595, 1603, C=C.

Mass Spec: Meas. Mass: 435. 2338; Calc. Mass 435. 2341.

To a solution of 1 - (6′ - methoxycarbonyl - n - butyl) - 2 - (3″ - hydroxy - 3″ - methyl - 4″ - phenoxy - n - butyl) - 4 - methyl - 1,2,4 - triazolidine - 3,5 - dione (1.11 g, 0.0026 mol) in methanol (40 ml) was added a 10% aqueous solution of sodium carbonate (8 ml) and the mixture boiled at reflux for 18 hrs. The reaction mixture was cooled and evaporated *in vacuo*. The residue was dissolved in water (40 ml) and extracted with ether (3×50 ml). The aqueous phase was then acidified with 10% hydrochloric acid and re-extracted with ethyl acetate (3×60 ml). The combined extracts were washed with saturated sodium chloride solution (2×100 ml), dried ($Na_2SO_4$), filtered and the filtrate concentrated *in vacuo* to afford 1 - (6′ - carboxy - n - hexyl) - 2 - (3″ - hydroxy - 3″ - methyl - 4″ - phenoxy - n - butyl) - 4 - methyl - 1,2,4 - triazolidine - 3,5 - dione (1.1 g) as a gum.

Analysis: C, 59.50; H, 7.75; N, 9.61%.
$C_{21}H_{31}N_3O_6$ requires C, 59.84; H, 7.41; N, 9.97%

N.m.r. ($CDCl_3$) $\tau$: 2.5—3.2 M, 5H, $C_6H_5$; 4.2 bs, 2H, $CO_2H$, OH; 6.0—6.7 bm, 6.15 s, 6H, $(N—CH_2)_2$, —$OCH_2Ph$; 6.94 S, 3H, —N—$CH_3$; 7.65 bt, 2H, $CH_2CO_2H$; 8.0—8.65 bm, 13H.

O O
|| ||
I.r ($cm^{-1}$): 2500—3450, $CO_2H$, OH; 1760, N—C; 1640—1740, N—C, —$CO_2$; 1603, C=C.

Mass. Spec: Meas. mass. 421. 2191; Calc. Mass, 421. 2212.

Example 5

Preparation of 1-(6′-carboxyhexyl)-2-(4″,4″-dimethyl-3″-hydroxy-8″-methoxyoctyl)-4-methyl-1,2,4-triazolidine-3,5-dione

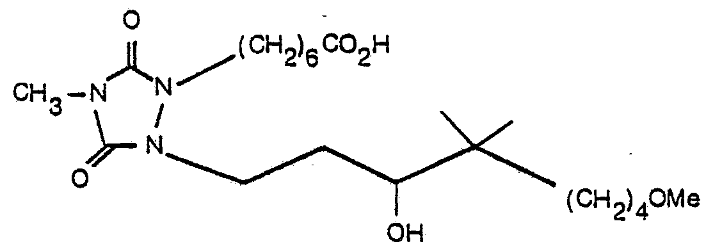

To a solution of 4 - methyl - 1,2,4 - triazolidine - 3,5 - dione (5.0 g, 0.0435 mol) in dimethylformamide (30 ml) was added portionwise sodium hydride as an 80% dispersion in mineral oil (1.44 g, 0.048 mol). The resultant mixture was stirred at 50°C under $N_2$ for 1 hr and then a solution of 4,4 - dimethyl - 8 - methoxy - oct - 1 - en - 3 - one (8.0 g, 0.0435 mol) in dimethylformamide (20 ml) was added dropwise. After 65 hr at 50°C the reaction mixture was cooled and poured into ice-water (100 ml), acidified with 10% hydrochloric acid and extracted with ethyl acetate (3×100 ml). The combined extracts were washed with water (2×100 ml), saturated sodium chloride solution (2×100 ml) and then dried ($Na_2SO_4$) and concentrated *in vacuo* to leave a gum (8.82 g). This gum was dissolved in diethyl ether (100 ml) and extracted with 2% aqueous sodium hydroxide solution (3×50 ml). The combined extracts were washed with diethyl ether (2×100 ml) and then acidified and re-extracted with ethyl acetate (3×100 ml). The combined extracts were washed with saturated sodium chloride solution (3×200 ml), dried ($Na_2SO_4$) and filtered, and the filtrate was concentrated *in vacuo* to give 2 - (4′,4′ - dimethyl - 8′ - methoxy - 3′ - oxo - n - octyl) - 4 - methyl - 1,2,4 - triazolidine - 3,5 - dione (5.45 g) as a gum.

N.m.r. ($CDCl_3$) τ: 2.1 bs, 1H, N—H; 5.18 t, 2H, N—$CH_2$; 6.45—6.70 m, 6.70 S, 5H, $CH_2OCH_3$; 6.95 S, 3H, N—$CH_3$; 7.15 t, $CH_2\overset{\overset{O}{|}}{C}$—; 8.2—9.0 bm, 8.88 S, 12H.

I.r (cm$^{-1}$): 3200, NH; 1760, C=O; 1700, N—$\overset{\overset{O}{\|}}{C}$.

To a solution of 2 - (4′,4′ - dimethyl - 8′ - methoxy - 3′ - oxo - n - octyl) - 4 - methyl - 1,2,4 - triazolidine - 3,5 - dione (4.485 g, 0.015 mol) in hexamethylphosphoramide (10 ml), was added sodium carbonate (2.0 g), sodium iodide (0.5 g) and a solution of ethyl - 7 - bromo - heptanoate (3.91 g, 0.016 mol) in hexamethylphosphoramide (10 ml). The resultant mixture was stirred for 6 days at room temperature and then poured into water (100 ml) and extracted with ethyl acetate (3×100 ml). The combined extracts were washed with water (3×100 ml) and saturated sodium chloride solution (2×100 ml) and then dried ($Na_2SO_4$) and filtered, and the filtrate was concentrated to leave an oil (7.75 g). This oil was chromatographed on silica gel (Merck Kieselgel 60, 250 g) using chloroform as eluant to afford an oil (2.93 g). This oil was dissolved in ethanol (50 ml) was sodium borohydride (0.268 g, 0.067 mol) and added portionwise, and the resultant mixture was stirred overnight at room temperature. The reaction was then concentrated *in vacuo*, dissolved in water (50 ml), acidified with 10% hydrochloric acid and extracted with ethyl acetate (3×50 ml). The combined extracts were washed with saturated sodium chloride solution (2×100 ml), dried ($Na_2SO_4$) and filtered, and the filtrate was concentrated *in vacuo* to afford a gum (2.92 g). This gum was chromatographed on silica gel (Merck Kieselgel 60, 90 g) to afford 1 - (6′ - ethoxycarbonyl - n - hexyl) - 2 - (4″,4″ - dimethyl - 3″ - hydroxy - 8″ - methoxy - n - octyl) - 4 - methyl - 1,2,4 - triazolidine - 3,5 - dione (1.87 g) as a gum.

N.m.r. ($CDCl_3$) τ: 5.79 q, 2H, $CO_2CH_2CH_3$; 6.07—7.32 bm, 7.32 S, 10H, $(N—CH_2)_2$, $CH$—OH, $CH_2$—$OCH_3$; 7.94 S, 3H, N—$CH_3$; 7.20 bs, 1H, —O—$H$; 7.22 bt, 2H, $CH_2CO_2Et$; 8.07—8.87 bm, 19H; 9.15 s, 6H.

I.r (cm$^{-1}$): 3450, —OH; 1770, N—$\overset{\overset{O}{\|}}{C}$; 1660—1740, N—$\overset{\overset{O}{\|}}{C}$, $CO_2Et$.

To a solution of 1 - (6′ - ethoxycarbonyl - n - hexyl) - 2 - 4″,4″ - dimethyl - 3″ - hydroxy - 8″ - methoxy - n - octyl) - 4 - methyl - 1,2,4 - triazolidine - 3,5 - dione (1.87 g) in ethanol (20

ml) was added a 10% aqueous solution of sodium carbonate (15 ml) and the mixture was boiled at reflux for 18 hr. The reaction mixture was cooled and evaporated *in vacuo*. The residue was dissolved in water (40 ml) and extracted with diethyl ether (3×50 ml). The aqueous phase was then acidified with 10% hydrochloric acid and re-extracted with ethyl acetate (3×60 ml). The combined extracts were washed with saturated sodium chloride solution (2×100 ml), dried ($Na_2SO_4$) and filtered, and the filtrate was concentrated *in vacuo* to afford 1 - (6′ - carboxy - n - hexyl) - 2 - (4″,4″ - dimethyl - 3″ - hydroxy - 8″ - methoxy - n - octyl) - 4 - methyl - 1,2,4 - triazolidine - 3,5 - dione (1.32 g) as a gum.

N.m.r. ($CDCl_3$) τ: 3.95 bs, 2H, —*OH*; 6.05—6.75 bm, 6.65 S, 10H, *CH*—OH, (N—$CH_2$)$_2$, $CH_2$—O—$CH_3$; 6.91 S, 3H, —N—$CH_3$; 7.65 bt, 2H, $CH_2CO_2H$; 8.0—8.9 bm, 16H, 9.13 bs, 6H.

I.r ($cm^{-1}$): 2500—3450, —OH; 1760, $N-\overset{\overset{O}{\|}}{C}$; 1640—1740, $N-\overset{\overset{O}{\|}}{C}$, $-CO_2$.

Example 6

Preparation of 4,4-dimethylhepta-1,6-dien-3-one

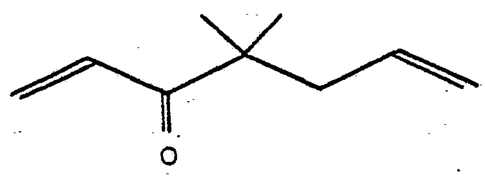

2,2 - Dimethylpent - 4 - enal was prepared as described by K. C. Braunsch, J. Amer. Chem. Soc., 1959, *81*, 3379.

To vinyl magnesium bromide, prepared from magnesium (13.4 g, 0.56 mol) and vinyl bromide (66.4 g, 0.62 mol) in tetrahydrofuran (100 ml), was added dropwise a solution of 2,2 - dimethylpent - 4 - en - 1 - al (46.3 g, 0.413 mol) in tetrahydrofuran (100 ml). The resultant mixture was stirred overnight at room temperature, poured into saturated ammonium chloride solution (200 ml) and extracted with diethyl ether (3×200 ml). The combined extracts were washed with saturated sodium chloride solution (2×200 ml), dried ($Na_2SO_4$), filtered and concentrated *in vacuo* to give an oil (52 g). This oil was distilled to afford 4,4 - dimethylhepta - 1,6 - diene - 3 - ol (45.4 g) b.p. 64—68°C/14 mm Hg. To a suspension of pyridinium chlorochromate (104.5 g, 0.486 mol) in dichloromethane (500 ml) stirred at room temperature was added rapidly a solution of 4,4 - dimethylhepta - 1,6 - dien - 3 - ol (45.4 g, 0.324 mol) in dichloromethane (70 ml) and cooled in an ice-bath. The reaction mixture was stirred for 2 hr at room temperature after subsidence of the initial exothermic reaction, diethyl ether (250 ml) was added, and the mixture was filtered. The filtrate was concentrated *in vacuo*, hydroquinone (100 mg) added and the residual oil was distilled to afford 4,4 - dimethylhepta - 1,6 - dien - 3 - one (24.7 g) bp. 58—62°C/(15 mm Hg) 2 kPa.

Example 7

Preparation of 2-(4′,4′-dimethyl-3′-oxo-hepta-6′-enyl)-4-methyl-1,2,4-triazolidine-3,5-dione

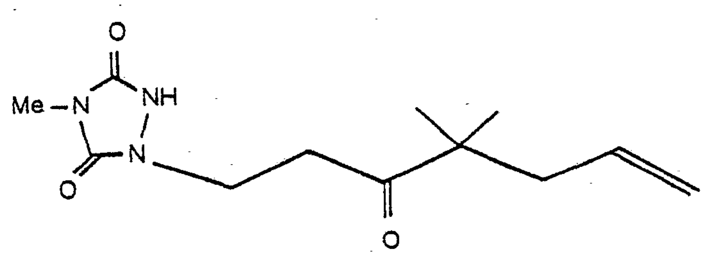

To a solution of 4 - methyl - 1,2,4 - triazolidine - 3,5 - dione (6.9 g, 0.06 mol) in dimethylformamide (70 ml) stirred at room temperature, under nitrogen, was added portionwise sodium hydride (1.98 g, 0.066 mol) as an 86% dispersion in mineral oil. The resultant mixture was stirred at 50°C for 6 hr and then a solution of 4,4 - dimethylhepta - 1,6 - dien - 3 - one (8.28 g, 0.06 mol) in dimethylformamide (10 ml) was added dropwise. After 3 days at 50°C, the reaction mixture was cooled and poured into water (200 ml), and the resultant mixture was acidified with 10% hydrochloric acid, and extracted with ethyl acetate (3×100 ml). The combined extracts were washed with water (3×100 ml) and saturated sodium chloride solution (2×100 ml) and then dried ($Na_2SO_4$) and filtered, and the filtrate was concentrated *in vacuo* to give an oil.

This oil was dissolved in diethyl ether (100 ml) and extracted with 2% aqueous sodium hydroxide solution (3×50 ml). The combined extracts were extracted with diethyl ether (2×100 ml), acidified and

re-extracted with ethyl acetate (3×100 ml). The combined ethyl acetate extracts were washed with saturated sodium chloride solution (3×100 ml) and then dried ($Na_2SO_4$) and filtered and the filtrate evaporated *in vacuo* to give 2 - (4′,4′ - dimethyl - 3′ - oxo - hept - 6′ - enyl) - 4 - methyl - 1,2,4 - triazolidine - 3,5 - dione as a gum (4.403 g).

N.m.r. ($CDCl_3$) $\tau$: 1.80 bs, 1H, —N—H; 3.96—4.62 m, 1H, 4.75—5.17 m, 2H; 6.18 t, 2H, —N—$CH_2$; 6.92 S, 3H, —N—$CH_3$; 7.12 t, 2H, $CH_2—\overset{O}{\overset{\|}{C}}$; 7.70 d, $CH$—; 8.85 S, 6H.

I.r ($cm^{-1}$): 2700—3300, N—H; 1760, $\overset{O}{\overset{\|}{C}}$—, N—$\overset{O}{\overset{\|}{C}}$; 1660—1720, N—$\overset{O}{\overset{\|}{C}}$.

## Example 8

Preparation of 1-(6′-ethoxycarbonyl-n-hexyl)-2-(4″,4″-dimethyl-3″-hydroxy-hept-6″-enyl)-4-methyl-1,2,4-triazolidine-3,5-dione

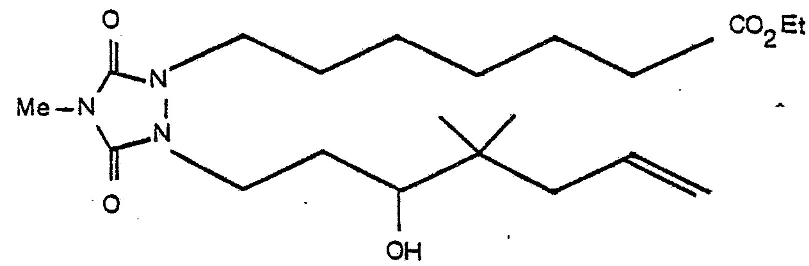

To a solution of 2 - (4′,4′ - dimethyl - 3′ - oxo - hept - 6′ - enyl) - 4 - methyl - 1,2,4 - triazolidine - 3,5 - dione (3.58 g 0.014 mol) in hexamethylphosphoramide (10 ml) was added sodium carbonate (2.0 g), sodium iodide (0.5 g) and a solution of ethyl - 7 - bromoheptanoate (4.12 g, 0.017 mol) in hexamethylphosphoramide (10 ml). The resultant solution was stirred at room temperature for 10 days and then poured into water (100 ml) and extracted with ethyl acetate (3×100 ml). The combined extracts were washed with water (3×100 ml) and saturated sodium chloride solution (2×100 ml) and then dried ($Na_2SO_4$) and filtered and the filtrate was evaporated *in vacuo* to leave a gum (5.98 g). This gum was dissolved in ethanol (75 ml), and sodium borohydride (0.611 g, 0.016 mol) was added portionwise. The resultant mixture was stirred overnight at room temperature and then concentrated *in vacuo*. The residue was dissolved in water (100 ml), acidified with 10% hydrochloric acid and extracted with ethyl acetate (3×100 ml). The combined extracts were washed with saturated sodium chloride solution (2×100 ml) and then dried ($Na_2SO_4$) and filtered and the filtrate concentrated *in vacuo* to leave a gum (5.89 g). This gum was chromatographed on silica gel (Kieselgel 60, 180 g) using chloroform as eluant to afford 1 - (6′ - ethoxycarbonyl - n - hexyl) - 2 - (4″,4″ - dimethyl - 3″ - hydroxy - hept - 6″ - enyl) - 4 - methyl - 1,2,4 - triazolidine - 3,5 - dione (2.0 g) as a gum.

N.m.r. ($CDCl_3$) $\tau$: 3.96—4.52 m, 1H, 4.88—5.1 m, 2H; 5.78 q, 2H; 6.28 m, 5H; 6.82 S, 3H, 7.34 bs, 1H; 7.45—8.2 bm, 8.65 t, 17H, 9.02 d, 6H.

I.r ($cm^{-1}$): 3450, —OH; 1760, N—$\overset{O}{\overset{\|}{C}}$; 1660—1740, N—$\overset{O}{\overset{\|}{C}}$, $CO_2Et$.

## Pharmacological data

### Broncho-dilation activity

The compound of Example 4 was examined for its ability to inhibit 5 - hydroxy - tryptamine or histamine induced bronchoconstriction in the anaesthetised artifically respired guinea-pig (Konzett-Rossler preparation).

The $ED_{50}$ value ($\mu$g/kg, i.v.) for inhibition of bronchoconstriction for the compound of Example 4 was 39.

### Anti-ulcer activity

Method

Anti-ulcer activity of the compound of Example 4 was assessed by the inhibition of indomethacin induced gastric damage in the rat according to the method of Eleghe (1974) Israeli J. Med. Sci. *10*. 1451. Rats were starved overnight, given 15 mg/kg indomethacin subcutaneously and sacrificed 4 hours later. Stomachs were reflated with n. saline, cut along the greater curvature pinned out and scored for gastric damage. Groups of 7 rats were used for each treatment and the test compound or

vehicle was administered 30 minutes prior to giving the indomethacin. Varying doses of test compound were administered orally and control groups receiving vehicle only were set up simultaneously. Mean values for each treatment were obtained and the Mann Witney test applied for significance of difference between the values obtained with the treatments. The results are shown in Table A.

TABLE A

| Dose mg/kg p.o. | % Inhibition of damage |
|---|---|
| 100 | 92 |
| 50 | 50 |
| 25 | 56 |

Anti-gastric secretory activity

The compounds were examined for their ability to inhibit pentagastrin-stimulated gastric acid secretion in the anaesthetised, perfused rat stomach preparation (Ghosh and Schild preparation, Parsons modification). The compounds were administered intra-venously. The results are shown in Table B.

TABLE B

| mg/kg i.v. | % Inhibition of secretion |
|---|---|
| 0.4 | 60 |
| 1.6 | 98 |

Toxicity

No toxic effects were observed in these tests.

**Claims**

1. A compound of the formula (I):

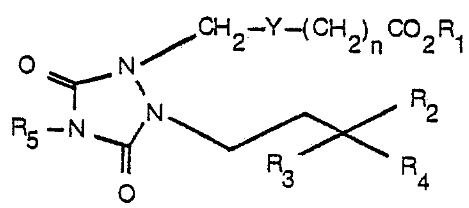

(I)

characterised in that:

n is 1 to 5;
Y is $—CH_2—CH_2$, —CH=CH— or —C≡C—
$R_1$ is hydrogen or $CO_2R_1$ represents an ester group in which the $R_1$ moiety contains from 1—12 carbon atoms;
$R_2$ is hydrogen, $C_{1-4}$ alkyl or phenyl;
$R_3$ is hydroxy or protected hydroxy;
$R_4$ is a group of the formula:

$$L—Q—R_{10}$$

in which:

Q is an oxygen atom, a —CH=CH— group or a —C≡C— group and
L is a $C_{1-9}$ alkylene group; and $R_{10}$ is $C_{1-4}$ alkyl, phenyl or naphthyl, any of which phenyl or naphthyl moieties may be substituted by one or more halogen, trifluoromethyl, $C_{1-6}$ alkyl, hydroxy, $C_{1-6}$ alkoxy, phenyl $C_{1-6}$ alkoxy or nitro groups, or, if Q is a —CH=CH— group, then $R_{10}$ may be a hydrogen atom; and $R_5$ is hydrogen or $C_{1-6}$ alkyl; and salts thereof.

2. A compound according to claim 1 of formula (II):

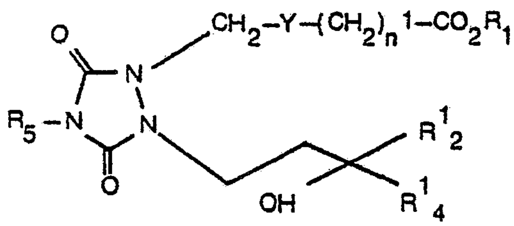

(II)

characterised in that:

Y, $R_1$ and $R_5$ are as defined in claim 1:
$n^1$ is 2, 3 or 4;
$R^1_2$ is hydrogen, methyl, ethyl or phenyl; and $R^1_4$ is a group of the formula:

$$L—O—R^1_{10}$$

in which

L is a $C_{1-9}$ alkylene group; and
$R^1_{10}$ is 1—4 alkyl;

and salts thereof.

3. A compound according to claim 1 of the formula (III):

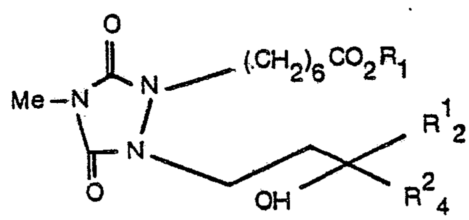

(III)

characterised in that:

$R_1$ is as defined in claim 1
$R^1_2$ is hydrogen, methyl or ethyl; and
$R^2_4$ is a group of the formula:

$$L^1—O—R^2_{10}$$

in which

$L^1$ is a $C_{4-8}$ alkylene group, and
$R^2_{10}$ is $C_{1-2}$ alkyl;

and salts thereof.

4. 1 - (6′ - carboxyhexyl) - 2 - (4″,4″ - dimethyl - 3″ - hydroxy - 8″ - methoxyoctyl) - 4 - methyl - 1,2,4 - triazolidine - 3,5 - dione.

5. A compound according to claim 1 of formula (IV):

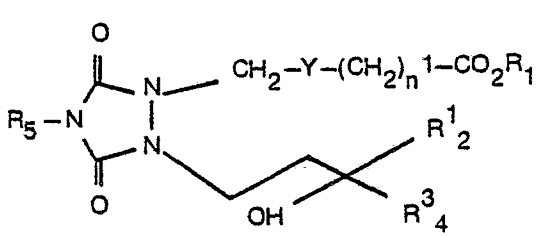

(IV)

characterised in that:

Y, $R_1$ and $R_5$ are as defined in claim 1;
n is 2, 3 or 4;
$R^1_2$ is hydrogen, methyl, ethyl or phenyl; and
$R^3_4$ is a group of formula (V):

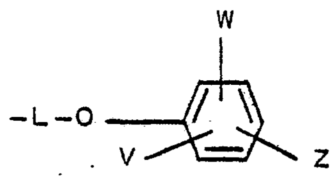

(V)

wherein:

L is as defined in claim 2 and V, W and Z are each hydrogen or fluorine, chlorine or bromine atoms, or $CF_3$, methyl, ethyl, *n*- or *iso*-propyl, methoxy, ethoxy, *n*- or *iso*-propoxy or nitro groups; and salts thereof.

6. 1 - (6′ - carboxyhexyl) - 2 - (3″ - hydroxy - 3″ - methyl - 4″ - phenoxybutyl) - 4 - methyl - 1,2,4 - triazolidine - 3,5 - dione.

7. A compound according to claim 1 of formula (VI):

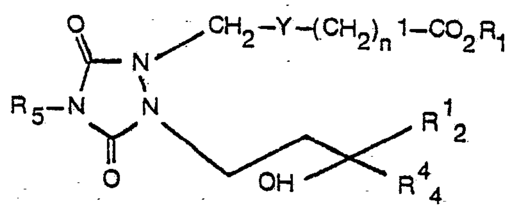

(VI)

characterised in that:

Y, $n^1$, $R_1$, $R^1_2$ and $R_5$ are as defined in claim 1; and $R^4_4$ is a group of the formula

$$—L—Q^1—R^3_{10}$$

in which

L is as defined in formula (I);
$Q^1$ is a —CH=CH— group or a —C≡C— group;
$R^3_{10}$ is $C_{1-4}$ alkyl, or if $Q^1$ is a —CH=CH— group, then $R^3_{10}$ may be a hydrogen atom; and salts thereof.

8. A compound according to claim 1 of formula (VII):

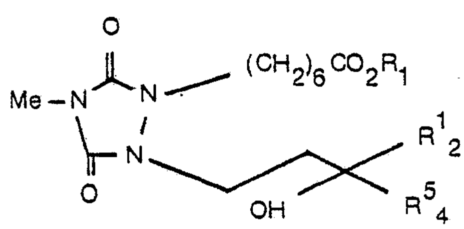

(VII)

characterised in that:

$R_1$ is as defined in claim 1;
$R^1_2$ is hydrogen, methyl or ethyl;
$R^5_4$ is a group of the formula

$$L^3—Q^1—R^4_{10}$$

in which

$L^3$ is a $C_{1-4}$ alkylene group
$Q^1$ is a group —CH=CH— or a group —C≡C—;
$R^4_{10}$ is $C_{1-2}$ alkyl or may be hydrogen if $Q^1$ is a —CH=CH— group; and salts thereof.

9. 1 - (6′ - ethoxycarbonylhexyl) - 2 - (4″,4″ - dimethyl - 3″ - hydroxyhept - 6″ - enyl) - 4 - methyl - 1,2,4 - triazolidine - 3,5 - dione.

10. A compound according to claim 1 of formula (VIII):

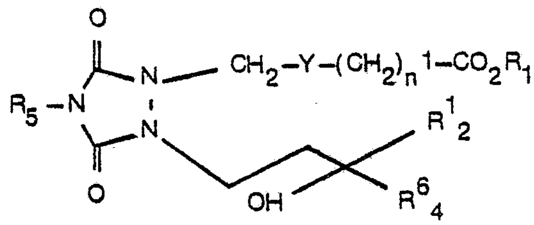

(VIII)

characterised in that:

Y, $R_1$ and $R_5$ are as defined in claim 1;
$n^1$ is 2, 3 or 4;
$R^1{}_2$ is hydrogen, methyl, ethyl or phenyl;
$R^6{}_4$ is a group of formula (IX):

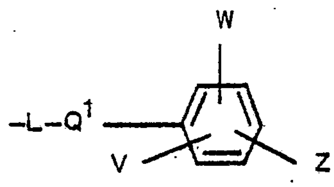

(IX)

wherein:

L and $Q^1$ are as defined in formula (VI); and V, W and Z are each hydrogen, fluorine, chlorine or bromine atoms, or $CF_3$, methyl, ethyl, *n-* or *iso-* propoxy or nitro groups, and salts thereof.

11. A pharmaceutical composition characterised in that it comprises a compound of the formula (I) as defined in claim 1 and a pharmaceutically acceptable carrier.

12. A process for the preparation of a compound of the formula (I) as defined in claim 1, characterised by either

a) reacting a compound of formula (X):

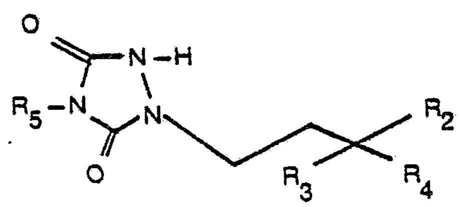

(X)

wherein:

$R_2$, $R_3$, $R_4$ and $R_5$ are as defined in claim 1, with a compound of formula (XI):

$$T{-}CH_2{-}Y{-}(CH_2)_nCO_2R_1 \qquad (XI)$$

wherein T is a group readily displaced by a nucleophile, and Y, n and $R_1$ are as defined in claim 1.

b) reacting a compound of the formula (XII):

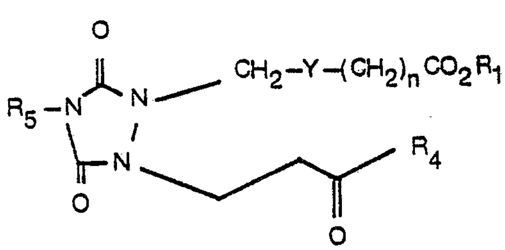

(XII)

wherein:

Y, n, $R_1$, $R_4$ and $R_5$ are as defined in claim 1; with a reducing agent to give a corresponding compound of the formula (I) wherein $R_2$ is hydrogen and $R_3$ is hydroxy, or with a $C_{1-4}$ alkyl or phenyl, Grignard reagent or $C_{1-4}$ alkyl or phenyl metallic complex to give a corresponding compound of the formula (I) wherein $R_2$ is $C_{1-4}$ alkyl or phenyl, and $R_3$ is hydroxy; and then optionally protecting the $R_3$ hydroxy moiety, or

c) when $R_5$ in the compound of formula (I) is not a hydrogen atom, reacting a compound of the formula (XIII)

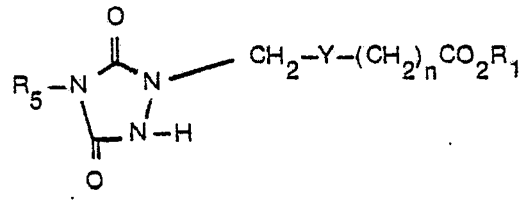

(XIII)

wherein:

Y, n, $R_1$ and $R_5$ are as defined in claim 1, with a compound of formula (XIV):

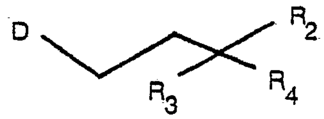

(XIV)

wherein the variables are as defined in claim 1.

**Patentansprüche**

1. Eine Verbindung der Formel (I):

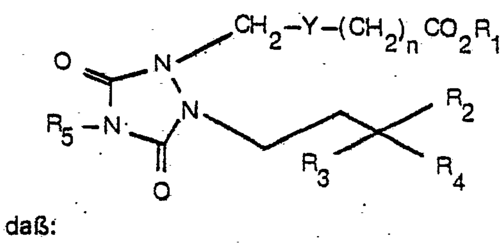

(I)

dadurch gekennzeichnet, daß:

n 1 bis 5 ist,
Y $-CH_2-CH_2$, $-CH=CH-$ oder $-C\equiv C-$ ist,
$R_1$ ein Wasserstoffatom ist oder $CO_2R_1$ eine Estergruppe bedeutet, in der der Rest $R_1$ 1 bis 12 Kohlenstoffatome enthält,
$R_2$ ein Wasserstoffatom, eine $C_{1-4}$-Alkyl- oder Phenylgruppe ist,
$R_3$ eine Hydroxylgruppe oder eine geschützte Hydroxylgruppe ist,
$R_4$ eine Gruppe der Formel:

$$L-Q-R_{10}$$

ist, in der:

Q ein Sauerstoffatom, eine $-CH=CH-$Gruppe oder eine $-C\equiv C-$ Gruppe ist und
L eine $C_{1-9}$-Alkylengruppe ist und $R_{10}$ eine $C_{1-4}$-Alkyl-, Phenyl- oder Naphthylgruppe ist, wobei jeder der Phenyl- oder Naphthylreste mit einem oder mehreren Halogenatomen, Trifluormethyl-, $C_{1-6}$-Alkyl-, Hydroxyl-, $C_{1-6}$-Alkoxy-, Phenyl-$C_{1-6}$-alkoxy- oder Nitro-Gruppe n substituiert sein kann, oder, wenn Q eine $-CH=CH$-Gruppe ist, $R_{10}$ ein Wasserstoffatom sein kann, und $R_5$ ein Wasserstoffatom oder eine $C_{1-6}$-Alkylgruppe ist, und Salze derselben.

2. Eine Verbindung gemäß Anspruch 1 der Formel (II):

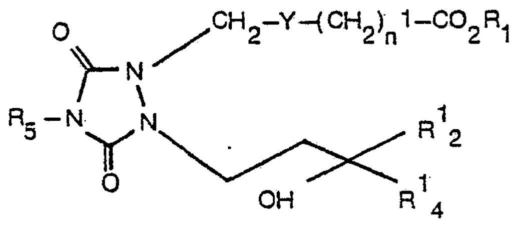

(II)

dadurch gekennzeichnet, daß:

Y, $R_1$ und $R_5$ wie in Anspruch 1 definiert sind,
$n^1$ 2, 3 oder 4 ist,

$R^1_2$ ein Wasserstoffatom, eine Methyl-, Äthyl- oder Phenyl- gruppe ist und $R^1_4$ eine Gruppe der Formel:

$$L—O—R^1_{10}$$

ist in der

L eine $C_{1-9}$-Alkylengruppe ist und
$R^1_{10}$ eine $C_{1-4}$-Alkylgruppe ist, und Salze derselben.

3. Eine Verbindung gemäß Anspruch 1 der Formel (III):

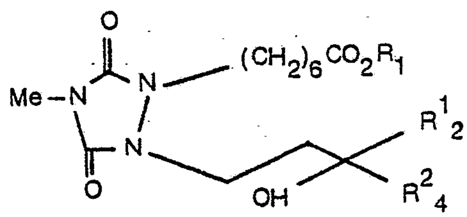

(III)

dadurch gekennzeichnet, daß

$R_1$ wie in Anspruch 1 definiert ist,
$R^1_2$ ein Wasserstoffatom, eine Methyl- oder Äthylgruppe ist und
$R^2_4$ eine Gruppe der Formel:

$$L^1—O—R^1_{10}$$

ist, in der

$L^1$ eine $C_{4-8}$-Alkylengruppe ist und
$R^2_{10}$ eine $C_{1-2}$-Alkylgruppe ist, und
Salze derselben.

4. 1 - (6′ - Carboxyhexyl) - 2 - (4″,4″ - dimethyl - 3″ - hydroxy - 8″ - methoxyoctyl) - 4 - methyl - 1,2,4 - triazolidin - 3,5 - dion.

5. Eine Verbindung gemäß Anspruch 1 der Formel (IV):

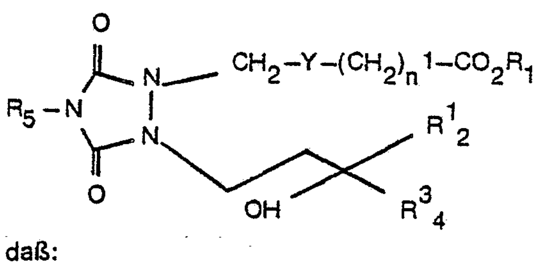

(IV)

dadurch gekennzeichnet, daß:

Y, $R_1$ und $R_5$ wie in Anspruch 1 definiert sind,
n 2, 3 oder 4 ist,
$R^1_2$ ein Wasserstoffatom, eine Methyl-, Äthyl- oder Phenyl-gruppe ist und
$R^3_4$ eine Gruppe der Formel (V) ist:

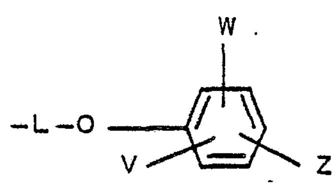

(V)

in der:

L wie in Anspruch 2 definiert ist und V, W und Z je ein Wasserstoff- oder Fluor-, Chlor- oder Bromatom oder eine $CF_3$—, Methyl-, Äthyl-, n- oder iso-Propyl-, Methoxy-, Äthoxy-, n- oder iso-Propoxy- oder Nitrogruppe ist, und Salze derselben.

6. 1 - (6′ - Carboxyhexyl) - 2 - (3″ - hydroxy - 3″ - methyl - 4″ - phenoxybutyl) - 4 - methyl - 1,2,4 - triazolidin - 3,5 - dion.

7. Eine Verbindung gemäß Anspruch 1 der Formel (VI):

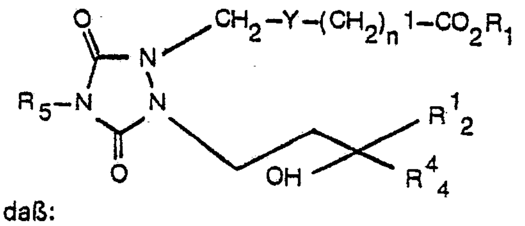

(VI)

dadurch gekennzeichnet, daß:

Y, $n^1$, $R_1$, $R^1_2$ and $R_5$ wie in Anspruch 1 definiert sind und $R^4_4$ eine Gruppe der Formel

$$—L—Q^1—R^3_{10}$$

ist, in der

L wie in Formel (I) definiert ist,
$Q^1$ eine —CH=CH— Gruppe oder eine —C≡C-Gruppe ist,
$R^3_{10}$ eine $C_{1-4}$-Alkylgruppe ist, oder, wenn $Q^1$ eine —CH=CH-Gruppe ist, $R^3_{10}$ ein Wasserstoffatom sein kann, und Salze derselben.

8. Eine Verbindung gemäß Anspruch 1 der Formel (VII):

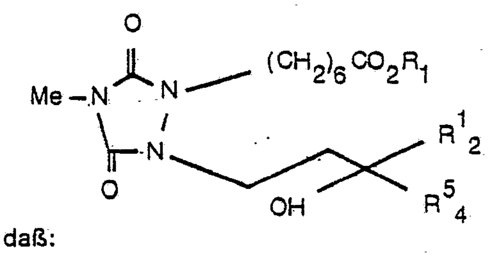

(VII)

dadurch gekennzeichnet, daß:

$R_1$ wie in Anspruch 1 definiert ist,
$R^1_2$ ein Wasserstoffatom, eine Methyl- oder Äthylgruppe ist,
$R^5_4$ eine Gruppe der Formel

$$L^3—Q^1—R^4_{10}$$

ist, in der

$L^3$ eine $C_{1-4}$-Alkylengruppe ist,
$Q^1$ eine —CH=CH-Gruppe oder eine —C≡C-Gruppe ist,
$R^4_{10}$ eine $C_{1-2}$-Alkylgruppe ist oder ein Wasserstoffatom sein kann, wenn $Q^1$ eine —CH=CH-Gruppe ist, und Salze derselben.

9. 1 - (6″ - Äthoxycarbonylhexyl) - 2 - (4″,4″ - dimethyl - 3″ - hydroxyhept - 6″ - enyl) - 4 - methyl - 1,2,4 - triazolidin - 3,5 - dion.

10. Eine Verbindung gemäß Anspruch 1 der Formel (VIII):

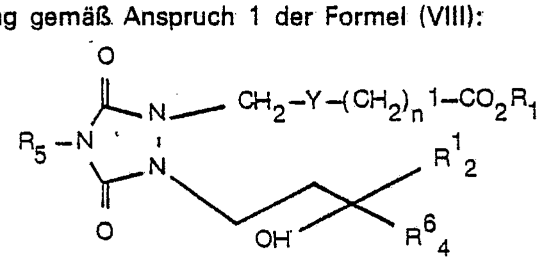

(VIII)

dadurch gekennzeichnet, daß:

Y, $R_1$ und $R_5$ wie in Anspruch 1 definiert sind,
$n^1$ 2, 3 oder 4 ist,
$R^1_2$ ein Wasserstoffatom, eine Methyl-, Äthyl- oder Phenyl-gruppe ist
$R^6_4$ eine Gruppe der Formel (IX) ist:

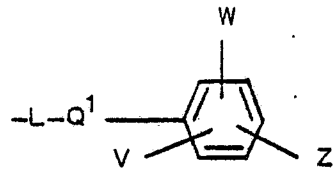

(IX)

in der:

L und $Q^1$ wie in Formel (VI) definiert sind und V, W und Z je ein Wasserstoff-, Fluor-, Chlor- oder Bromatom, oder eine $CF_3$-, Methyl-, Äthyl-, n- oder iso-Propoxy- oder Nitro-gruppe ist, und Salze derselben.

11. Eine pharmazeutische Zusammensetzung, dadurch gekennzeichnet, daß sie eine Verbindung der Formel (I), wie in Anspruch 1 definiert, und einen pharmakologisch verträglichen Träger enthält.

12. Ein Verfahren zur Herstellung einer Verbindung der Formel (I), wie in Anspruch 1 definiert, dadurch gekennzeichnet, daß entweder

a) eine Verbindung der Formel (X):

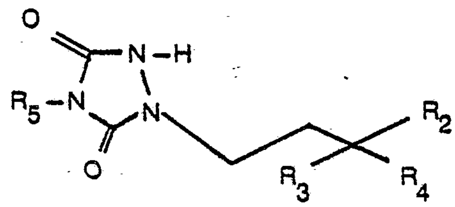

(X)

in der:

$R_2$, $R_3$, $R_4$ und $R_5$ wie in Anspruch 1 definiert sind, mit einer Verbindung der Formel (XI) umgesetzt wird:

$$T—CH_2—Y—(CH_2)_nCO_2R_1$$

in der T eine durch eine nukleophile Gruppe XI leicht verdrängbare Gruppe ist, und Y, n und $R_1$ wie in Anspruch 1 definiert sind, oder

b) eine Verbindung der Formel (XII):

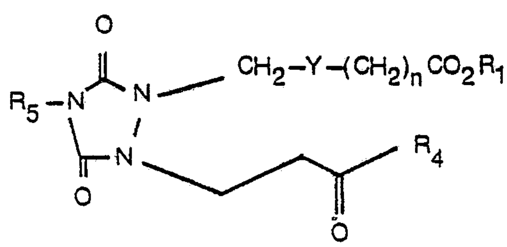

(XII)

in der:

Y, n, $R_1$, $R_4$ und $R_5$ wie in Anspruch 1 definiert sind, mit einem reduzierenden Mittel zu einer entsprechenden Verbindung der Formel (I) umgesetzt wird, in der $R^2$ ein Wasserstoffatom und $R_3$ eine Hydroxylgruppe ist, oder mit einem $C_{1-4}$-Alkyl- oder Phenyl-Grignard-Reagenz oder $C_{1-4}$-Alkyl- oder Phenyl-Metallkomplex zu einer entsprechenden Verbindung der Formel (I), umgesetzt wird in der $R_2$ eine $C_{1-4}$-Alkyl- oder Phenylgruppe ist und $R_3$ eine Hydroxylgruppe ist, und anschließend gegebenenfalls die $R_3$-Hydroxylgruppe geschützt wird, oder

c) wenn $R_5$ in der Verbindung der Formel (I) nicht ein Wasserstoffatom ist, eine Verbindung der Formel (XIII):

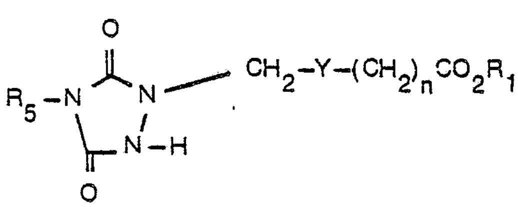

(XIII)

in der:

Y, n, $R_1$ und $R_5$ wie in Anspruch 1 definiert sind, mit einer Verbindung der Formel (XIV) umgesetzt wird:

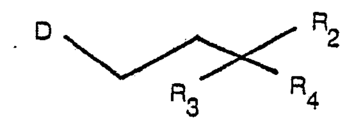

(XIV)

in der die Variablen wie in Anspruch 1 definiert sind.

**Revendications**

1. Composé de formule (I):

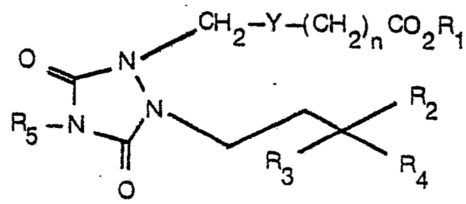

(I)

caractérisé en ce que:

n a une valeur allant de 1 à 5;
Y est $—CH_2—CH_2$, —CH=CH— ou C≡C—;
$R_1$ est de l'hydrogène ou bien $CO_2R_1$ représente un groupe ester dans lequel la fraction molaire $R_1$ contient de 1 à 12 atomes de carbone;
$R_2$ est de l'hydrogène ou un alcoyle en $C_{1-4}$ ou un phényle;
$R_3$ est un hydroxy ou un hydroxy protégé;
$R_4$ est un groupe de formule

$$L—Q—R_{10}$$

dans laquelle:

Q est un atome d'oxygène, un groupe —CH=CH— ou un groupe —C≡C— et
L est un groupe alcoylène en $C_{1-9}$; et $R_{10}$ est un groupe alcoyle en $C_{1-4}$, phényle ou naphtyle, l'une quelconque des fractions molaires phényle ou naphtyle pouvant être substituée par un ou plusieurs halogènes ou groupes trifluorométhyle, alcoyle en $C_{1-6}$, hydroxy, alcoxy en $C_{1-6}$, phényl-alcoxy ($C_{1-6}$) ou nitro; ou bien si Q est un groupe —CH=CH—, $R_{10}$ peut être un atome d'hydrogène; et $R_5$ est de l'hydrogène ou un alcoyle en $C_{1-6}$; et les sels de ce composé.

2. Composé suivant la revendication 1 de formule (II):

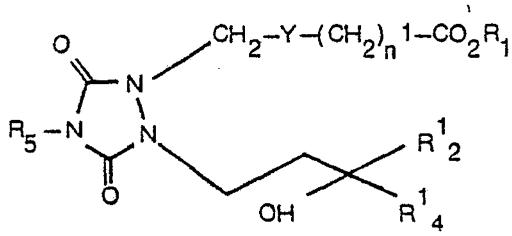

(II)

caractérisé en ce que

Y, $R_1$ et $R_5$ ont la même signification que dans la revendication 1;
$n^1$ est égal à 2, 3 ou 4;
$R^1_2$ est de l'hydrogène ou un groupe méthyle, éthyle ou phényle;
et $R^1_4$ est un groupe de formule:

$$L—O—R^1_{10}$$

dans laquelle:

L est un groupe alcoylène en $C_{1-9}$; et
$R^1_{10}$ est un alcoyle en $C_{1-4}$;

et les sels de ce composé.

3. Composé suivant la revendication 1 de formule (III):

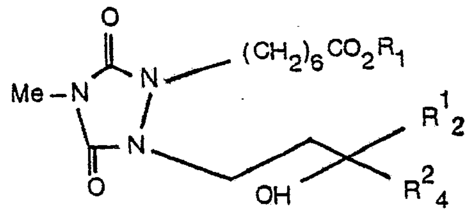

(III)

caractérisé en ce que

$R_1$ a la même signification que dans la revendication 1;
$R^1_2$ est de l'hydrogène, un méthyle ou un éthyle; et
$R^2_4$ est un groupe de formule

$$L^1—O—R^2_{10}$$

dans laquelle:

$L^1$ est un groupe alcoylène en $C_{4-8}$, et
$R^2_{10}$ est un alcoyle en $C_{1-2}$;
et les sels de ce composé.

4. 1 - (6' - Carboxyhexyl) - 2 - (4",4" - diméthyl - 3" - hydroxy - 8" - méthoxyoctyl) - 4 - méthyl - 1,2,4 - triazolidine - 3,5 - dione.

5. Composé suivant la revendication 1 de formule (IV):

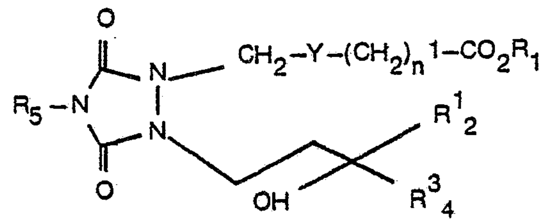

(IV)

caractérisé en ce que

Y, $R_1$ et $R_5$ on la même signification que dans la revendication 1;
$n^1$ est égal à 2, 3 ou 4;
$R^1_2$ est de l'hydrogène ou un groupe méthyle, éthyle ou phényle; et
$R^3_4$ est un groupe de formule (V):

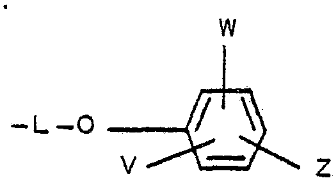

(V)

dans laquelle:

L a la même signification que dans la revendication 2 et V, W et Z sont chacun de l'hydrogène ou des atomes de fluor, de chlore ou de brome ou des groupes $CF_3$, méthyle, éthyle, n- ou iso-propyle, méthoxy, éthoxy, n- ou iso-propoxy ou nitro; et les sels de ce composé.

6. 1 - (6' - Carboxyhexyl) - 2 - (3" - hydroxy - 3" - méthyl - 4" - phénoxybutyl) - 4 - méthyl - 1,2,4 - triazolidine - 3,5 - dione.

7. Composé suivant la revendication 1 de formule (VI):

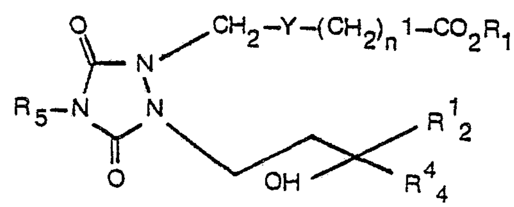

(VI)

caractérisé en ce que

Y, $n^1$, $R^1_2$ et $R_5$ ont la même signification que dans la revendication 1; et $R^4_4$ est un groupe de formule:

$$—L—Q^1—R^3_{10}$$

dans laquelle:

$Q^1$ est un groupe —CH=CH— ou un groupe —C≡C—;
$R^3_{10}$ est un alcoyle en $C_{1-4}$ ou bien si $Q^1$ est un groupe —CH=CH—, alors $R^3_{10}$ peut être un atome d'hydrogène; et les sels de ce composé.

8. Composé suivant la revendication 1 de formule (VII):

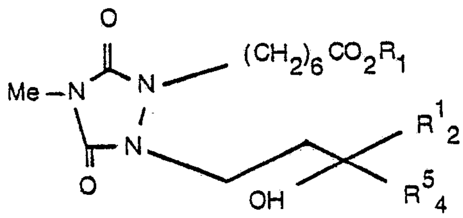

(VII)

caractérisé en ce què

$R_1$ a la même signification que dans la revendication 1;
$R^1_2$ est de l'hydrogène ou un groupe méthyle ou éthyle;
$R^5_4$ est un groupe de formule:

$$L^3—Q^1—R^4_{10}$$

dans laquelle:

$L^3$ est une groupe alcoylène en $C_{1-4}$;
$Q^1$ est un groupe —CH=CH— ou un groupe —C≡C—;
$R^4_{10}$ est un alcoyle en $C_{1-2}$ ou peut être de l'hydrogène si $Q^1$ est un groupe —CH=CH—; et les sels de ce composé.

9. 1 - (6' - Ethoxycarbonylhexyl) - 2 - (4'',4'' - diméthyl - 3'' - hydroxyhept - 6'' - ényl) - 4 - méthyl - 1,2,4 - triazolidine - 3,5 - dione.

10. Composé suivant la revendication 1 de formule (VIII):

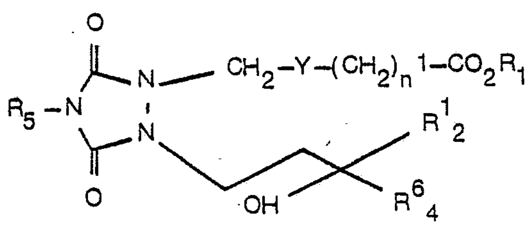

(VIII)

caractérisé en ce que

Y, $R_1$ et $R_5$ ont la même signification que dans la revendication 1;
$n^1$ est égal à 2, 3 ou 4;
$R^1_2$ est de l'hydrogène ou un groupe méthyle, éthyle ou phényle;
$R^6_4$ est un groupe de formule (IX):

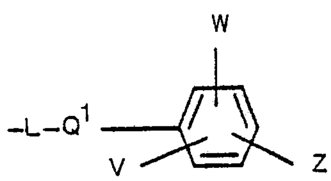

(IX)

dans laquelle:

L et $Q^1$ ont la même signification que dans la formule (VI); et V, W et Z sont chacun de l'hydrogène ou un atome de fluor, de chlore ou de brome ou des groupes $CF_3$, méthyle, éthyle, n- ou iso-propoxy ou nitro; et les sels de ce composé.

11. Composition pharmaceutique, caractérisé en ce qu'elle renferme un composé de formule (I) suivant la revendication 1 et un véhicule ou excipient pharmaceutiquement acceptable.

12. Procédé pour la préparation d'un composé de formule (I) suivant la revendication 1, caractérisé en ce que:

a) on fait réagir un composé de formule (X):

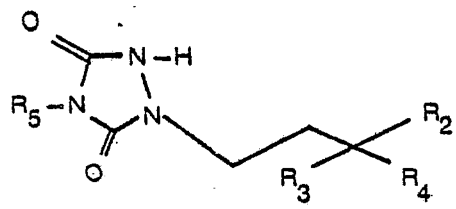

(X)

dans laquelle

$R_2$, $R_3$, $R_4$ et $R_5$ sont tels que définis dans la revendication 1, avec un composé de formule (XI):

$$T—CH_2—Y—(CH_2)_nCO_2R_1 \qquad (XI)$$

dans laquelle T est un groupe aisément déplacé par un nucléophile, et Y, n et $R_1$ sont tels que définis dans la revendication 1;

b) on fait réagir un composé de formule (XII):

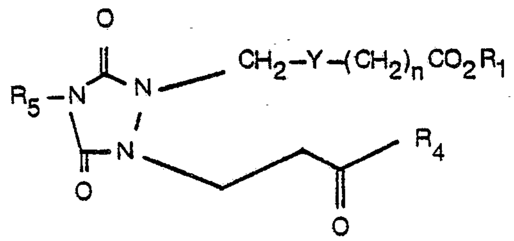

(XII)

dans laquelle:

Y, n, $R_1$, $R_4$ et $R_5$ ont la même signification que dans la revendication 1, avec un agent réducteur, pour donner un composé correspondant de formule (I) dans laquelle $R_2$ est de l'hydrogène et $R_3$ est un hydroxy, ou avec un réactif de Grignard à alcoyle en $C_{1-4}$ ou phényle, ou un complexe métallique d'alcoyle en $C_{1-4}$ ou de phényle pour donner un composé correspondant de formule (I) dans laquelle $R_2$ est un alcoyle en $C_{1-4}$ ou un phényle et $R_3$ est un hydroxy, puis éventuellement ou protégé la fraction molaire $R_3$ hydroxy, ou

c) quand $R_5$ dans le composé de formule (I) n'est pas un atome d'hydrogène, on fait réagir un composé de formule (XIII):

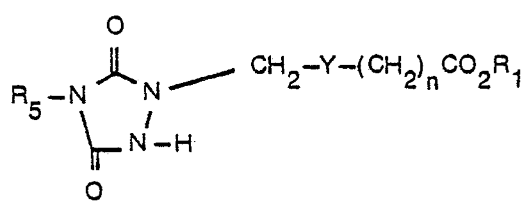

(XIII)

dans laquelle

Y, n, $R_1$ et $R_5$ sont tels que définis dans la revendication 1 avec un composé de formule (XIV):

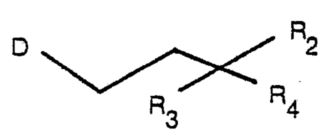

(XIV)

dans laquelle les variables sont telles que définies dans la revendication 1.